# EUROPEAN PATENT SPECIFICATION

(11) **EP 2 928 871 B1**
(45) Date of publication and mention of the grant of the patent: **01.02.2017**
(21) Application number: 13821730.2
(22) Date of filing: 06.12.2013
(51) Int. Cl.: C07D 233/64, A61K 31/4172, A61P 35/00

(54) **HISTIDINYLATED CATIONIC AMPHIPHILES, PROCESS FOR PREPARATION THEREOF AND THEIR LIPOSOMAL FORMULATION**
HISTIDINYLIERTE KATIONISCHE AMPHIPHILE, VERFAHREN ZU IHRER HERSTELLUNG UND IHRE LIPOSOMALE FORMULIERUNG
AMPHIPHILES CATIONIQUES HISTIDINYLÉS, PROCÉDÉ DE PRÉPARATION ASSOCIÉ ET FORMULATION EN LIPOSOME ASSOCIÉE

(30) Priority: 07.12.2012 IN DE37672012
(43) Date of publication of application: 14.10.2015
(73) Proprietor: Council of Scientific & Industrial Research, New Delhi 110 001 (IN)
(72) Inventor: GARU, Arup, Hyderabad 500 607 (IN); MOKU, Gopikrishna, Hyderabad 500 607 (IN); AGAWANE, Sachin, Barad, Hyderabad 500 607 (IN); CHAUDHURI, Arabinda, Hyderabad 500 607 (IN)
(74) Representative: Fairbairn, Angus Chisholm
(86) International application number: PCT/IN2013/000751
(87) International publication number: WO 2014/087429

(56) References cited:
- US-A1- 2008 306 153
- US-A1- 2012 100 205

## Description

### FIELD OF THE INVENTION

The present invention relates to histidinylated cationic amphiphile compounds. The present invention also relates to methods for preparing a novel series of histidinylated cationic amphiphiles. The invention provides novel compositions containing the said cationic amphiphiles which possess anti-cancer activity. The invention also relates to liposomal formulations of the cationic amphiphiles. The liposomal formulations exhibit anti-proliferative activity and are useful for treatment of cancer in human and animal body. The cationic amphiphiles disclosed herein show enhanced cellular apoptosis through the apoptotic signal transduction pathway. The area of medical science that is likely to benefit most from the present invention is cancer therapy.

### BACKGROUND OF THE INVENTION

The dreadful disease of cancer results from uncontrolled cell growth. A distinguishing feature of cancer cells is their capability of growing without the help of any external growth inducing signals. Cancer cells are often loosely attached to the surrounding cells or the extracellular matrix and thus they usually divide more rapidly than normal cells. Angiogenesis, formation of new blood vessels from pre-existing blood vessels, occurs mainly during embryonic development, wound healing, female reproductive cycle, haemangiomas, diabetic retinopathy, age related muscular degeneration, psoriasis, gingivitis, rheumatoid arthritis and during solid tumour growth (Carmeliet, P. et al. Nature 2000, 14, 6801). When tumor cells grow beyond a size of 1-2 mm³, they secrete enzymes and growth signals (cytokines) which in turn direct nearer blood vessels to form sub-vessels directed toward the growing tumor cells (Folkman, J. et al. Science 1987, 235, 442-447; Folkman, J. et al. Science 1989, 243, 1490-1493). Such newly formed tumor vasculatures (neovasculature) ensures food and nutrient supply to the growing tumor cells. This is why anti-angiogenic cancer therapy is a promising approach for combating growing tumours.

Amphiphiles possessing imidazole functionalities have found elegant biotechnological applications in the past particularly in the field of non-viral gene delivery. For instance, toward protecting genetic materials from hydrolytic digestion within the endosomes, Wolff and his co-workers pioneered the design of efficient pH-sensitive cationic transfection lipids containing weakly basic lysosomotropic imidazole head-groups (Nature Biotechnol, 1996, 14, 760-764). In the area of cationic polymer mediated gene delivery, remarkable transfection efficiency was achieved by Midoux and coworkers through covalent grafting of endosome-disrupting multiple histidine functionalities in the molecular architecture of cationic polymer (Adv Drug Deliv Rev 2001; 53: 75-94). It was demonstrated previously that covalent grafting of single histidine functionality in the head-group region imparts high gene transfer efficacy to cationic amphiphiles presumably due to their enhanced bio membrane fusogenicity (Kumar, V. et al. Gene. Ther. 2003, 10, 1206-1215; Karmali, P.P. et al. Bioconjugate Chemistry 2006, 17, 159-171). However, use of histidinylated cationic amphiphiles as anti-cancer compound has not been reported before. The present invention discloses procedures for synthesizing aspartic and glutamic acid based novel histitdinylated cationic amphiphiles and their therapeutic potential as novel anti-cancer compounds. The present invention also discloses the tumor growth inhibition properties of the liposomal formulations of these novel histidinylated cationic amphiphiles in intra-tumoral mice model.

### OBJECT OF THE INVENTION

The main object of the present invention is to provide histidinylated cationic amphiphile compound of formula I.

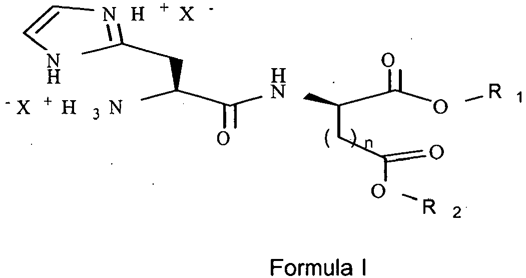

Another object of the present invention is to provide a process for preparing a novel series of histidinylated cationic amphiphile of formula I.

One more object of the present invention is to provide novel compositions containing the said cationic amphiphile which possess anti-cancer activity.

Further object of the invention is to provide liposomal formulations of the cationic amphiphile which exhibit anti-proliferative activity and are useful for treatment of cancer.

Still another object of the invention is to provide compound which shows better cytotoxicity than those of similar liposomal formulations of the commercially available hydrophobic anti-cancer drug, in cancerous cells.

One more object of the invention is to provide cationic amphiphile which showed enhanced cellular apoptosis through the apoptotic signal transduction pathway.

### SUMMARY OF THE INVENTION

Accordingly, the present invention provides a histidinylated cationic amphiphile compound of formula I. 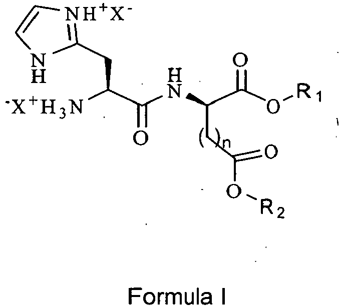
wherein R1 and R2 are each independently hydrogen or a lipophilic moiety provided both R1 and R2 are not hydrogen at the same time;
X is chlorine or bromine;
n is selected from 1-2 ;
   wherein the said lipophilic moiety is selected from the group consisting of C₈₋₂₄ alkyl, monounsaturated, diunsaturated and triunsaturated alkenyl.

In another embodiment of the invention, compound is selected from the group consisting of compound of formula A and B 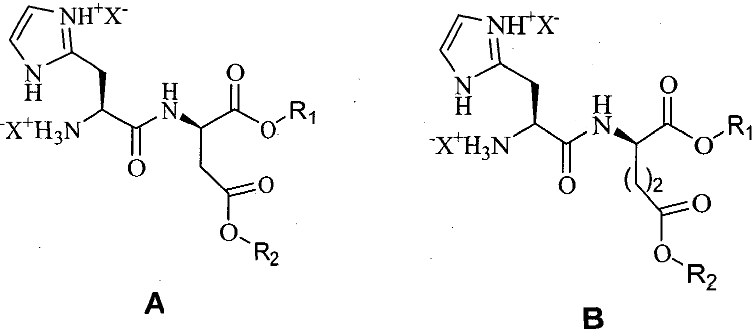
Wherein R1 and R2 are each independently hydrogen or a lipophilic moiety provided both R1 and R2 are not hydrogen at the same time;
X is chlorine or bromine;
   wherein the said lipophilic moiety is selected from the group consisting of C₈₋₂₄ alkyl, monounsaturated, diunsaturated and triunsaturated alkenyl.

In yet another embodiment of the present invention, the compound is selected from the group consisting of:
(i):2-((S)-2-ammonio-3-((R)-1,4-bis(hexadecyloxy)-1,4-dioxobutan-2-ylamino)-3-oxopropyl)-1 H-imidazol-3-iumchloride (HD16),
(ii):2-((S)-2-ammonio-3-((R)-1,4-bis(octadecyloxy)-1,4-dioxobutan-2-ylamino)-3-oxopropyl)-1 H-imidazol-3-iumchloride (HD18),
(iii):2-((S)-2-ammonio-3-((R)-1,5-bis(hexadecyloxy)-1,5-dioxopentan-2-ylamino)-3-oxopropyl)-1 H-imidazol-3-iumchloride (HE16), and
(iv):2-((S)-2-ammonio-3-((R)-1,5-bis(octaadecyloxy)-1,5-dioxopentan-2-ylamino)-3-oxopropyl)-1 H-imidazol-3-iumchloride (HE18).

In another embodiment of the present invention, the compound induces apoptosis and inhibits angiogenesis in both tumor endothelial cell and tumor cells.

In another embodiment of the present invention the compound produce cytotoxic effect in cancer cells at concentration ranging between 10 to 17 micromolar.

One more embodiment of the present invention is to provide compound which inhibits bCL-2 and NF-kB based on cell survival pathways.

Still another embodiment of the present invention is to provide compound which inhibits 70-90% tumour growth at a dose ranging from 3.0 to 3.5 mg/kg Accordingly, the present invention provides a process for the preparation of compound of formula I, the process comprising of following steps;
(a) coupling of aliphatic alcohol with N Boc protected derivatives in the presence of a coupling agent and racemization suppressing agent in a polar aprotic solvent followed by purification to obtain compound of formula II ; 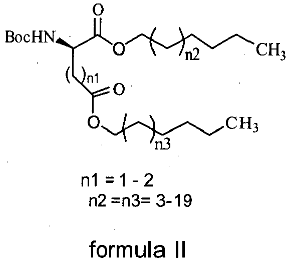
(b) deprotecting the compound of formula II obtained from step (a) using TFA(trifluoro acetic acid) as deprotecting agent and filtration to obtain compound of formula III; 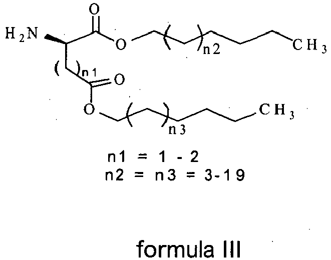
(c) adding N Boc protected histidine to a compound of formula III in presence of a raceimization suppressing agent and coupling agentin a polar aprotic solvent followed by purification to obtain compound of formula IV ; and 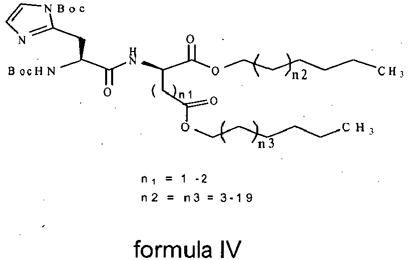
(d) reacting the compound of formula IV obtained from step (c) in presence of a polar aprotic solvent and TFA as deprotecting agent followed by ion exchange chromatography to obtain the compound of general formula I.

In another embodiment of the invention, the aliphatic alcohol is selected from the group consisting of saturated or unsaturated alcohol having 8-24 carbon atoms.
In another embodiment of the present invention, the polar aprotic solvent is selected from the group consisting of dichloromethane, dimethyl formamide, dimethylsulphoxide, pyridine and triethyl amine.
In still another embodiment of the present invention, the coupling agent is selected from group consisting of EDCl (1-ethyl-3-(3-dimethylaminopropyl) carbodiimide) and DCC (13-Dicyclohexyl carbodiimide).
In yet another embodiment of the present invention, the racemization suppressing agent is selected from group consisting of HOBt (1-Hydroxybenzotriazole) and HOAt.(1-hydroxy-7-azabenzotriazole).

In another embodiment, the present invention, provides a liposomal formulation comprising of a histindinylated cationic amphiphile of formula I a co-lipid and a polyanionic compound optionally along with physiological acceptable additive.

In another embodiment of the invention, the cationic amphiphiles is either alone or in combination with helper lipids.

In another embodiment of the present invention, the co lipid are selected from the group consisting of phosphatidylethanolamine, phosphatidylphosphocholine, phosphatidylglycerol, cholesterol, 1,2-syn-dioleyolglycerophosphoethanolamine (DOPE) and 1,2-syn-dioleyolglycerophosphocholine (DOPC).

In still another embodiment of the invention, the helper lipid is *N*,*N*- Di-*n*-hexadecyl-*N*-[2-guanidinyl]ethyl-*N*- methylammonium Chloride (Q16TG).

In still another embodiment of the present invention, the molar ratio of cationic amphiphiles:co-lipid:helper lipid rangies from 1:1:1 to 3:2:1.

In yet another embodiment of the present invention, the polyanionic compound is an anionic synthetic phospholipid.

One more embodiment of the present invention, the physiological acceptable additives is selected from a group consisting of saline and 5% glucose solution.

In still another embodiment of the invention, the formulation shows cellular cytotoxicity in the range of 2 to 30 micro molar.

The present invention also provides a method for preparation of liposomal formulation, the method comprises of following steps;
(a) dissolving a cationic amphiphile of formula I, a co-lipid, and a helper lipid in the mole ratio ranging from 1:1:1 to 3:2:1 in a mixture of methanol and chloroform followed by evaporation of the solvent in presence of a thin flow of moisture free nitrogen gas to obtain lipid film;
(b) drying the lipid film obtained from step (a) under high vacuum;
(c) hydrating the dried lipid film in sterile deionized water; and
(d) vortexing the resulting mixture obtained in previous step (c), to remove any adhering lipid film, sonicating the vortexed mixture in a bath sonicator at room temperature to produce multilamellar vesicles (MLVs) and sonicating the resulting MLVs with Ti-probe sonicator to produce clear translucent small unilamellar vesicles (SUVs).

In yet another embodiment, the co lipid is selected from the group comprising phosphatidylethanolamine, phosphatidylphosphocholine, phosphatidylglycerol, cholesterol. 1,2-syn-dioleyolglycerophosphoethanolamine (DOPE) and 1,2-syn-dioleyolglycerophosphocholine (DOPC).

In one another embodiment, the helper lipid is *N,N-* Di-*n*-hexadecyl-*N*-[2-guanidinyl]ethyl-N- methylammonium Chloride (Q16TG).

In yet another embodiment, the ratio of chloroform and methanol used in step (a) ranges from 1:1 to 4:1.

In one another embodiment, the drying of lipid film is done for a period between 6-8 hours.

In yet another embodiment, the hydration of lipid film is done for a minimum of 12 hours.

### BRIEF DESCRIPTION OF ACCOMPANYING DRAWINGS

Scheme 1 is a schematic representation of the synthetic procedures used for the preparation of aspartic acid based histidinylated cationic amphiphiles.
Scheme 2 is a schematic representation of the synthetic procedures used for the preparation of glutamic acid based histidinylated cationic amphiphiles.

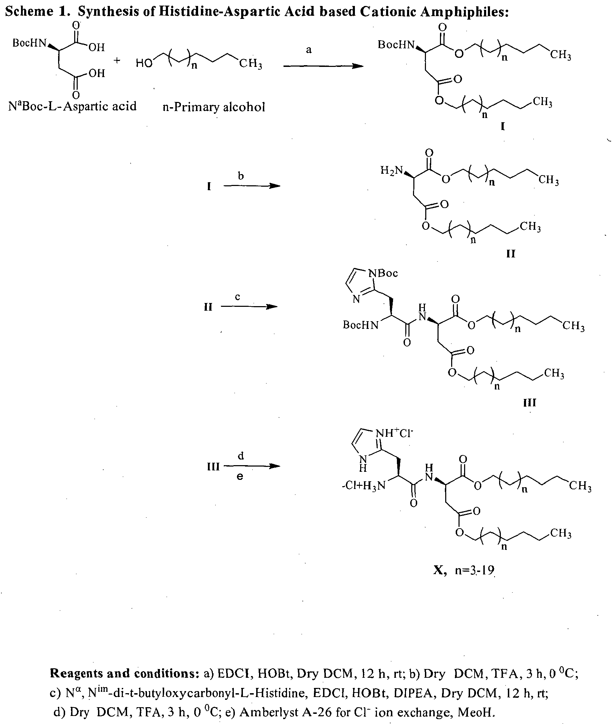

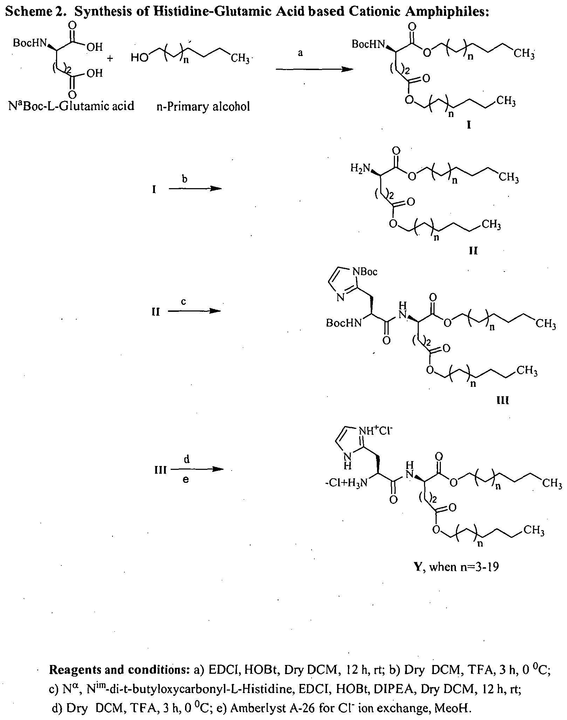

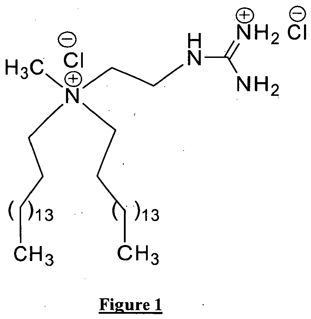

Figure 1 shows structure of the helperlipid *N*,*N*-Di-*n*-hexadecyl-*N* [2-guanidinyl] ethyl-*N*-methylammonium Chloride (Q16TG).

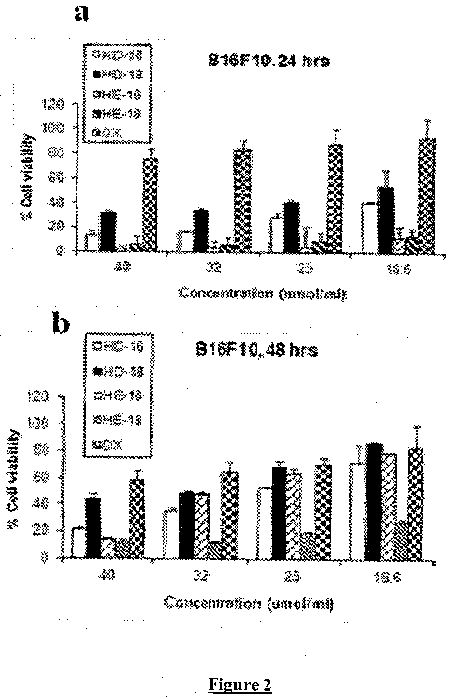

Figure 2: Summarizes the cytotoxic effect of lipids HD-16, HD-18, HE-16, HE-18 & Dexamethasone (DX) in combination with co lipid such as 1,2-syn-dioleyolglycerophosphoethanolamine (DOPE) and 1,2-syn-dioleyolglycerophosphocholine (DOPC) and helper lipid such as Q16TG on B16F10 cells (mouse melanoma tumor cells) in the concentration range 16-40 µmol/mL using MTT assay. The absorption obtained with reduced formazan with cells in the absence of lipids was taken to be 100. The assay was done after 24 (A) and 48 (B) hrs of the treatment.

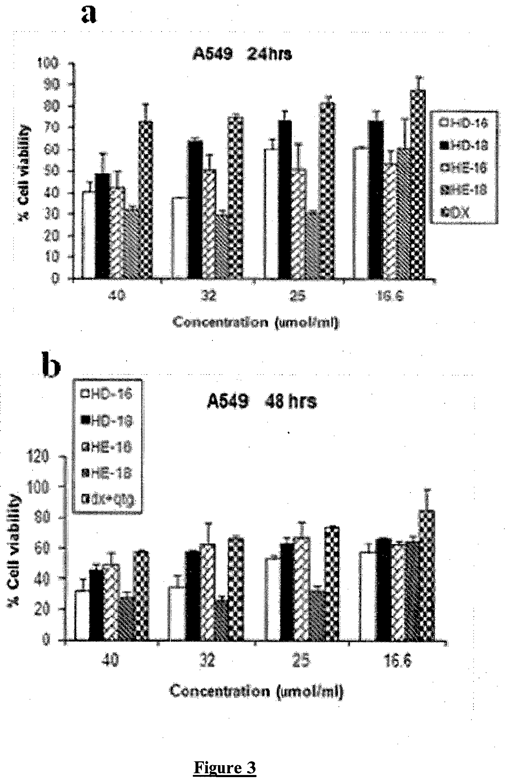

Figure 3: Summarizes the cytotoxic effect of lipids HD-16, HD-18, HE-16, HE-18 & DX i in combination withco-lipid such as DOPC and helper lipid such as Q16TG on A549 cells (human lung carcinoma cells) in the concentration range 16-40 µmol/mL using MTT assay. The absorption obtained with reduced formazan with cells in the absence of lipids was taken to be 100. The assay was done after 24 (A) and 48 (B) hrs of the treatment.

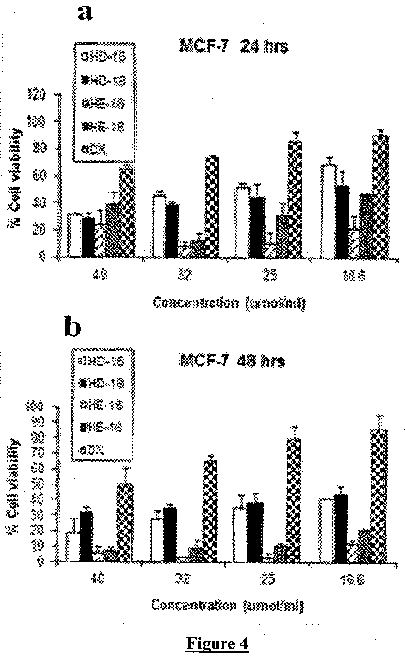

Figure 4: Summarizes the cytotoxic effect of lipids HD-16, HD-18, HE-16, HE-18 & DX present in combination with DOPC as co-lipid and Q16TG as helper lipid on MCF 7 cells (mouse breast cancer cells) in the concentration range 16-40 µmol/mL using MTT assay. The absorption obtained with reduced formazan with cells in the absence of lipids was taken to be 100. The assay was done after 24 (A) and 48 (B) hrs of the treatment.

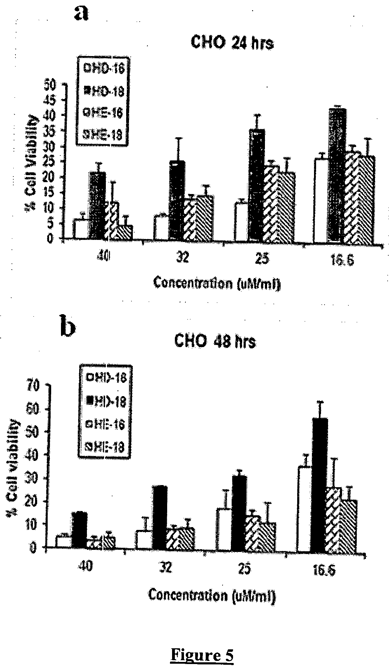

Figure 5: Summarizes the cytotoxic effect of lipids HD-16, HD-18, HE-16, HE-18 & DX present in combination with DOPC as co-lipid and Q16TG as helper lipid on CHO cells (Chinese hamstar ovarian cancer cells) in the concentration range 16-40 µmol/mL using MTT assay. The absorption obtained with reduced formazan with cells in the absence of lipids was taken to be 100. The assay was done after 24 (A) and 48 (B) hrs of the treatment.

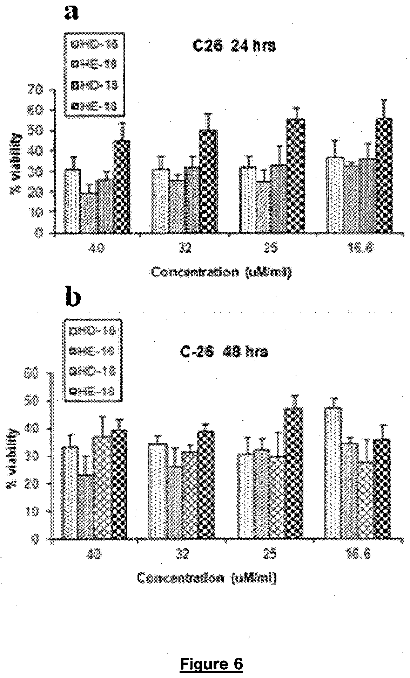

Figure 6: Summarizes the cytotoxic effect of lipids HD-16, HD-18, HE-16, HE-18 present & DX in combination with DOPC as co-lipid and Q16TG as helper lipid on C-26 cells (mouse colon carcinoma cells)in the concentration range 16-40 µmol/mL using MTT assay. The absorption obtained with reduced formazan with cells in the absence of lipids was taken to be 100. The assay was done after 24 (A) and 48 (B) hrs of the treatment.

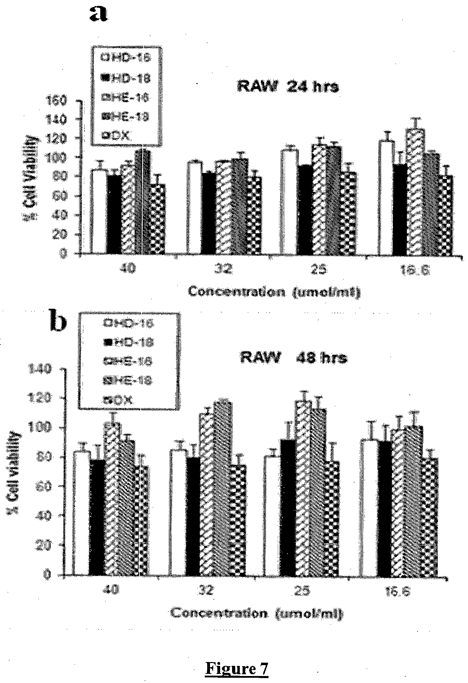

Figure 7: Summarizes the cytotoxic effect of lipids 1, 2, 3, 4 HD-16, HD-18, HE-16, HE-18 present & DX in combination with DOPC as co-lipid and Q16TG as helper lipid on non-cancerous healthy mouse macrophage cells (Raw 264.7 cells) in the concentration range 16-40 µmol/mL using MTT assay. The absorption obtained with reduced formazan with cells in the absence of lipids was taken to be 100. The assay was done after 24 (A) and 48 (B) hrs of the treatment.

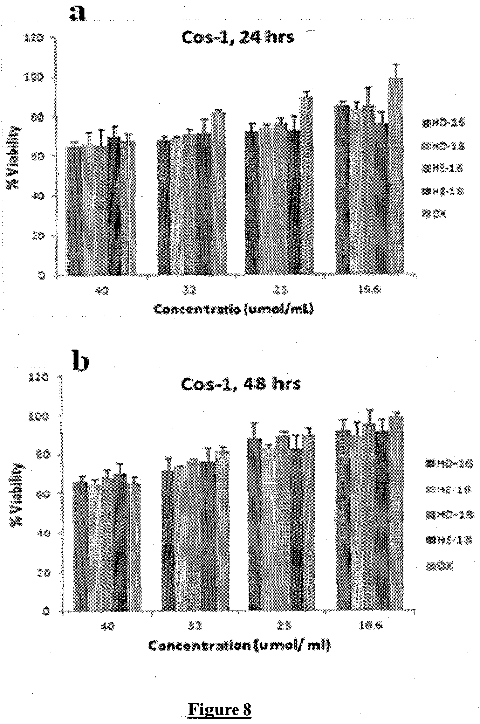

Figure 8: Summarizes the cytotoxic effect of lipids HD-16, HD-18, HE-16, HE-18 & DX present in combination with DOPC as co-lipid and Q16TG as helper lipid on non-cancerous healthy COS-1 cells in the concentration range 16-40 umol/mL using MTT assay. The absorption obtained with reduced formazan with cells in the absence of lipids was taken to be 100. The assay was done after 24 (A) and 48 (B) hrs of the treatment.

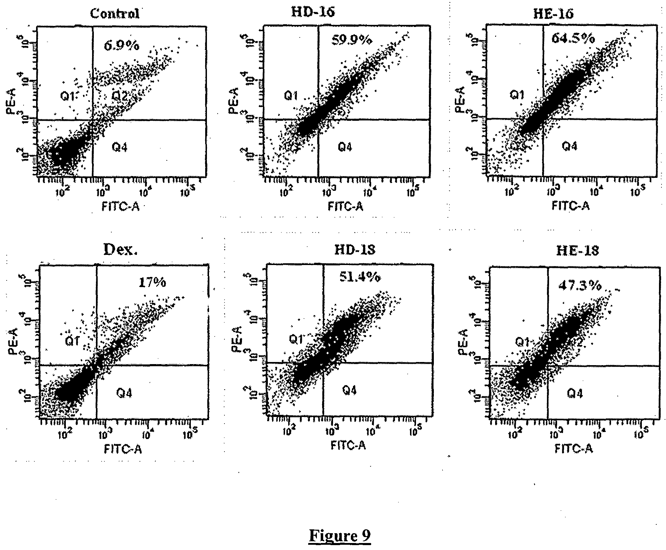

Figure 9 Describes apoptosis inducing property of histidinylated lipidsHD-16, HD-18, HE-16, HE-18 & DX on B16F10 cancer cells. Significant increase of both FITC (fluorescence isothio cyanate) and PE (phycoerythrin) signal in histidinylated lipid treated B16F10 cells confirm apoptosis inducing property of these lipids after 24 hrs.
In a flow cytometric quadrant (FITC vs PE), the lower left =(LL) panel represents normal cells and lower right (LR) panel represents early apoptotic cells which increase FITC signal only. Whereas upper right (UR) panel represents late apoptotic cells which increases both FITC and PE signaling and upper left (UL) panel represents necrotic cell only which increases PE signal only. A significant shift of events toward UR, UL and LR panel in case of histidinylated lipid treated cells compared to untreated cells doubly confirm the apoptosis inducibily property of these four lipids.

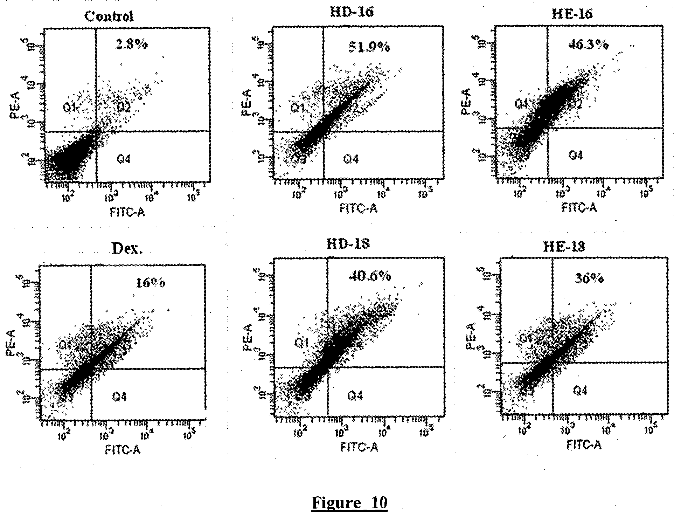

Figure 10 Describes apoptosis inducible property of histidinylated lipids HD-16, HD-18, HE-16, HE-18 & DX on A549 cancer cells. Significant increase of both FITC and PE signal in histidinylated lipid treated A549 cells confirm apoptosis inducibility property of these lipids after 24 hrs. In a flow cytometric quadrant (FITC vs PE), the lower left (LL) panel represents normal cells and lower right (LR) panel represents early apoptotic cells which increase FITC signal only. Whereas upper right (UR) panel represents late apoptotic cells which increases both FITC and PE signaling and upper left (UL) panel represents necrotic cell only which increases PE signal only. A significant shift of events toward UR, UL and LR panel in case of histidinylated lipid treated A549 cells compared to untreated cells doubly confirm the apoptosis inducibily property of these four lipids.

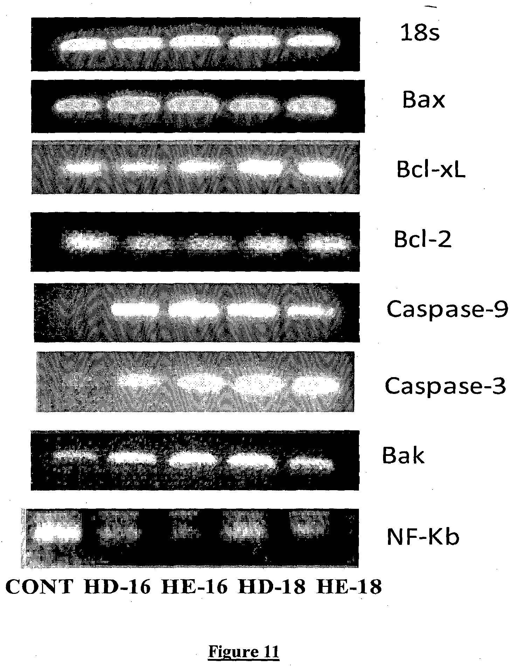

Figure 11 Describes reverse transcription-PCR analysis of (BAX, BAK, Caspase 3 & 9) and anti-apoptotic genes (Bcl-2, Bcl-xL & NF-*k*B) in A549 cells after 24 h treatment with histidinylated lipids. A549 cells were treated with liposome of histidinylated lipids in combination with DOPC as co-lipid and Q16TG as helper lipid in the concentration 25 µM/ml. After 4 h the medium was discarded and 3 mL of DMEM medium (Sigma) containing 10% fetal bovine serum was added to each well and left for 24 h. After 24 h, mRNAs were extracted from all the cells, cDNA was synthesized by Reverse transcription reaction and amplified by Polymerase Chain Reaction and finally resolved in 2% agarose gel.

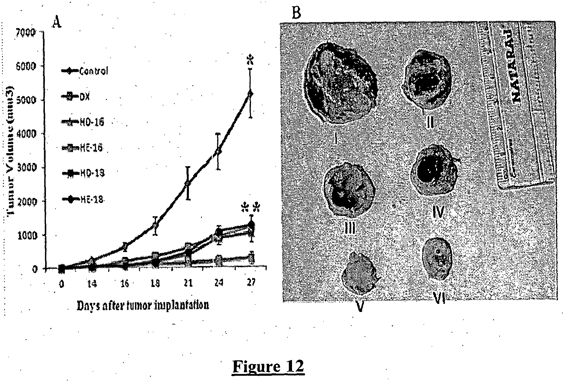

Figure 12 summarizes tumour growth inhibition properties of the histidinylated lipids HD-16 (1), HD-18 (2), HE-16 (3), HE-18 (4) & Dexamethasone (Dx) a. 6-8 weeks old female C57BL/6 mice (each weighing 20-22 g) bearing aggressive melanoma tumours (produced by subcutaneous injections of ∼1 x 10⁵ B16F10 cells in 100 µL Hank's buffer salt solution, HBSS into the left flanks on day 0) were randomly sorted into six groups and each group (n = 5) was injected s.c. with cationic liposome HD-16 (light green) ;
HE-16 (deep green); HD-18 (black); HE-18 (light blue); dexamethazone (red) only Q16TG & DOPC liposomal formulation (control, deep blue) on day 14, 16, 18, 21 and 24. Tumour volumes (V = 1/2.ab2 where, a = maximum length of the tumour and b = minimum length of the tumour measured perpendicular to each other) were measured with a slide calipers for up to 23 days. Results represent the means +/- SD (for n = 5 tumours) (*P < 0.01 vs HD16 and **P <0.005 vs control); B. Representative samples of melanoma tumors excised on day 27. I. tumour treated with vehicle only; II. tumour treated with dexamethazone only; III. tumour treated with HE-18; IV. tumour treated with HD-18; V. tumour treated with HD-16; VI tumour treated with HE-16.

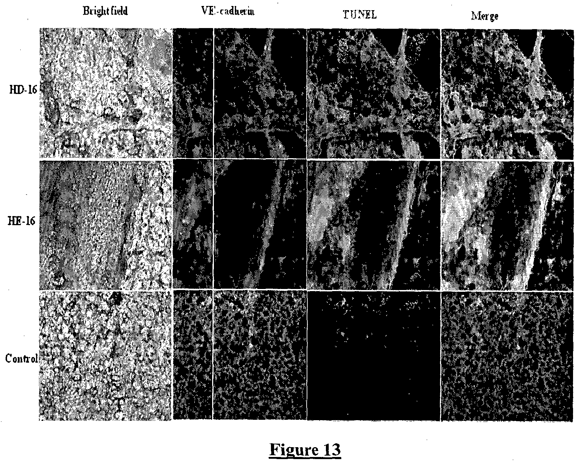

Figure 13 depicts tumor growth inhibition property of the histidinylated lipids are mediated through apoptosis of both tumor endothelial cells and tumor cells. The female C57 BL/6J mice were subcutaneously implanted with B16F10 melanoma cells (1.5 x 10⁵ cells) and the tumors were allowed to grow to ∼ 2000 mm³. The mice were injected s.c. with cationic liposome HD-16; HE-16; & only Q16TG & DOPC liposomal formulation (control) in three consecutive days. The mice were sacrificed 24 h post injection and the tumor was excised, cryosectioned, fixed and the fixed frozen sections were treated with TUNEL (*t*erminal deoxyuridine triphosphate nick-end *l*abeling, a widely used marker of apoptosis) assay kit for marking apoptotic cells (2^{nd} panels from the left, green). Subsequently, the same tumor cryosections were immunounostained with monoclonal antibody against VE-cadherin for marking tumor endothelial cells (3^{rd} panels from left, red). The 4^{th} panels (from the left) show superimposed images (yellow). The stained tumor slides were observed in the same positions under fluorescent microscope (10X magnification) using green and red filters. The 1^{st} panels from the left in both images (24 & 72 h) show the tissue architecture when observed in bright field. Bar = 2.0 µm.

### DETAILED DESCRIPTION OF THE INVENTION

The present invention discloses novel series of histidinylated cationic amphiphiles. It also relates to the process for preparing histidinylated cationic amphiphiles and more specifically relates to the process for preparing aspartic and glutamic acid based histidinylated cationic amphiphiles. The compounds of the present invention possess anti-proliferative activity such as anti-cancer activity and are useful in methods of treatment of various human and animal body cancers. The pharmaceutically active composition of the novel histidinylated cationic amphiphiles disclosed herein show enhanced cellular apoptosis (programmed cell death) in cancer cells through the apoptosis signal transduction pathway.

In a preferred embodiment of the invention, a histidinylated cationic amphiphile possesses formula I 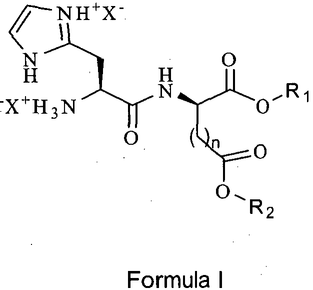 wherein
wherein R1 and R2 are each independently hydrogen or a lipophilic moiety provided both R1 and R2 are not hydrogen at the same time;
X is chlorine or bromine;
n is selected from 1-2 ;
Wherein said lipophilic moiety is selected from the group consisting of C₈₋₂₄ alkyl, monounsaturated, diunsaturated and triunsaturated alkenyl.

In a second preferred embodiment of the present invention, the disclosed cationic lipid with histidine head group is represented by structure 1 wherein R₁ = R₂ = n-hexadecyl, and X⁻ is a chloride ion.

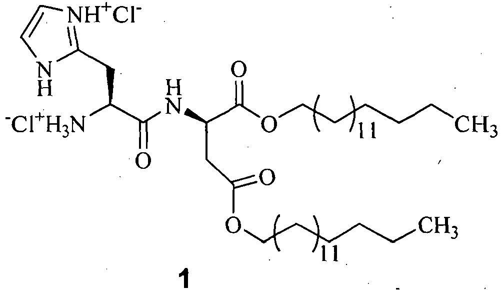

In another preferred embodiment of the present invention, the disclosed histidinylated cationic lipid is represented by structure 2 wherein R₁ = R₂ = n-octadecyl and X⁻ is a chloride ion.

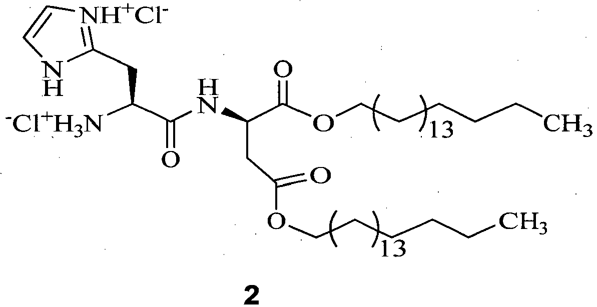

In yet another embodiment of the present invention, the glutamic acid based histidinylated cationic amphiphile is represented by structure 3 wherein R₁ = R₂ = n-hexadecyl, and X- is a chloride ion.

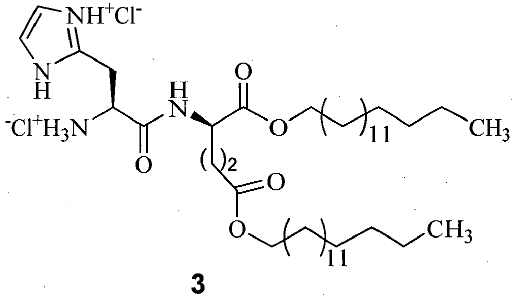

In yet another embodiment of the present invention, the glutamic acid-based cationic amphiphile with histidine head-group is represented by structure 4 wherein R₁ = R₂ = n-octadecyl and X⁻ is a chloride ion.

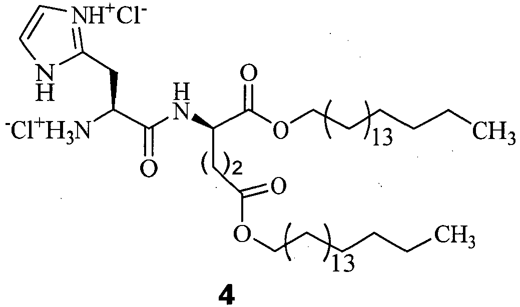

In still another embodiment of the invention, each of R₁ and R₂ in structure A is independently hydrogen or an aliphatic hydrocarbon chain provided both R1 and R2 are not hydrogen at the same time;.
In yet another embodiment of the invention, each of R₁ and R₂ groups in structure A is independently C₈₋₂₄ alkyl group), a mono-, di- or tri-unsaturated alkenyl.
In still another embodiment of the invention, R₁ and R₂ in structure A are the same and are C₈₋₂₄ mono-unsaturated alkenyl group.
In yet another embodiment of the invention, X in structure A is selected from the halogen group.
In another embodiment of the invention, the histidinylated cationic amphiphile are used in combination with helper lipids.
In another aspect of the invention, the co lipid are selected from the group consisting of phosphatidylethanolamine and phosphatidylglycerol.
In an embodiment of the invention, the formulations the co lipid are selected from group consisting of sterol group, a neutral phosphatidyl ethanolamine and neutral phosphatidyl choline.

In another embodiment the co lipid are preferentially selected from group consisting of 1,2-syn-dioleyolglycerophosphoethanolamine(DOPE) and 1,2-syn-dioleyolglycerophosphocholine (DOPC).

In another feature of the invention, the co-lipidsused is DOPC and helper lipid is *N,N-*Di-*n*-hexadecyl-*N*-[2-guanidinyl]ethyl-*N*-methylammonium Chloride (Q16TG).
In further aspect of the invention, the liposomal formulation, the molar ratio of the catioinic lipid to co lipid is in the range of 1:1:1 to 3:1:1.
In further embodiment of the invention, the preferred molar ratio of catioinic amphiphiles, co lipid and helper lipid is 1:1:1.
In another aspect of the invention, it shows cellular cytotoxicity, where the amount of cationic amphiphiles are in the range of 2 to 30 micro molar concentration.
In further embodiment of the invention, the said liposomalformulations are administered via cutaneous, sub-cutaneous, intradermal, nasal, intravenous, intramuscular, intraperitonial or pulmonary route.
In another important aspect of the invention, the said compound is useful in treating several cancers, comprising: lung cancer, melanoma, breast cancer, colon or ovarian cancer.
In further aspect of the invention, the said liposomal formulation of histidinylated lipids can induce apoptosis in both mouse and human cancerous cell line.
In another feature of the invention, the compound show enhanced cellular apoptosis through inhibition of BCL-2 (B-cell lymphoma 2) and activation of BAX (BCL-2 associated X proteins) signal transduction pathway.
In further embodiment of the invention, the compound shows enhanced cellular apoptosis through up regulating cysteine-dependent aspartate-directed protease3 (Caspase-3) & cysteine-dependent aspartate-directed protease9 (Caspase-9) signal transduction pathway.

In another feature of the invention, the compound shows enhanced cellular apoptosis through inhibiting pro-survival nuclear factor-kappa beta (NF-kB) signaling pathway.
In another important aspect of the invention, the said lipids can inhibit the tumor growth in animal body administered via cutaneous, sub-cutaneous, intradermal, intravenous, intramuscular, intraperitonial.

In another embodiment of the invention, the histidinylated lipid induces apoptosis in both tumor endothelial cell as well as tumor cells in tumor micro-environment.
In another feature of the invention, the presently disclosed compound inhibits angiogenesis by killing the tumor endothelial cells in tumor micro-environment.
In another embodiment of the invention, the compound is more efficient in anti-cancer activity than commercially available anti-cancer drug dexamethasone in same liposomal formulation.
The currently practiced cancer treatment modalities include surgery, radiation, chemotherapy or a combination of any of these treatments. Among these arsenals of treatment modalities, chemotherapy continues to be an indispensable approach for inoperable or metastatic cancers. The critical issue of drug resistance is reducing the efficacies of conventional chemotherapeutic drugs (e.g., cisplatin, thiotepa, chlorambucil, doxorubicin, paclitaxel, etc.). Multifunctional anticancer chemotherapeutic agents for cancer treatments have been reported in the past (Willmann, J. K. et. al. Nat. Rev. Drug. Discov. 2008, 7, 591).

Cationic lipids have received a lot of attention during the past 30 years as pharmaceutical carriers for its great potential to deliver many therapeutic drug as well as genes under the systemic setting. It was demonstrated previously that covalent grafting of single histidine functionality in the head-group region imparts high gene transfer efficacies to cationic amphiphiles presumably due to their enhanced biomembrane fusogenicity (Kumar, V. et al. Gene. Ther. 2003, 10, 1206-1215; Karmali, P.P. et al. Bioconjugate Chemistry 2006, 17, 159-171). However, use of histidinylated cationic amphiphiles as anti-cancer compound has not been reported before. The present invention discloses procedures for synthesizing aspartic and glutamic acid based novel histitdinylated cationic amphiphiles and their therapeutic potential as novel anti-cancer compounds. The present invention also discloses the tumor growth inhibition properties of the liposomal formulations of these novel histidinylated cationic amphiphiles in intra-tumoral mice model. The liposomal formulations of the cationic amphiphiles described herein exhibit anti-proliferative activity and are useful in methods of treatment of cancer in human and animal body. The cationic amphiphiles disclosed herein induce enhanced cellular apoptosis in various cancer cells and not in non-cancerous healthy cells. The present invention also discloses the tumor growth inhibition properties of the liposomal formulations of these novel histidinylated cationic amphiphiles in intra-tumoral mice model.

The distinctive novel structural features common to the cationic amphiphiles with histidine head-groups disclosed in the present invention include: (1) The presence of hydrophilic groups imidazole group of histidine located near the positively charged nitrogen, (2) The presence of aspartic and glutamic acid back bone in the molecular architectures of the presently disclosed cationic amphiphiles and (3) The non-polar long aliphatic long chain covalently linked to aspartic or glutamic acids through ester bond. The area of science that is likely to be benefited most from the present invention is the field of cancer therapy. According to the practice of the present invention, "cationic" means the positive charge is on a protonated nitrogen atom in α-amine group of histidine. Such cationic character is expected to enhance interaction between the cationic amphiphiles and the negatively charged biological cell membranes and thereby leading to enhanced cellular uptake of the presently disclosed anti-cancer molecules.

The cationic lipids with histidine head-groups disclosed herein have certain common structural and functional groups. As such, the compound is selected from group consisting of compound of formula A and B: 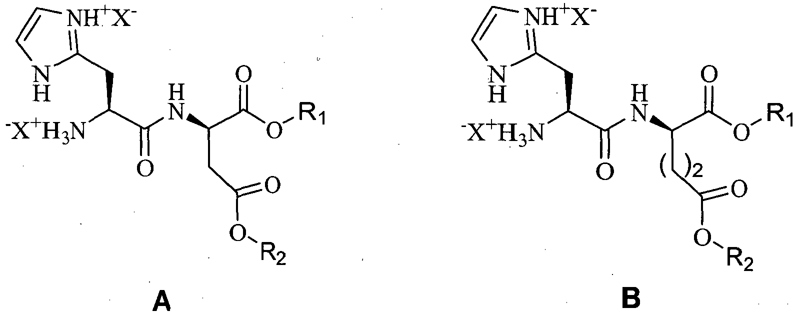 wherein
R1 and R2 are each independently hydrogen or a lipophilic moiety provided both R1 and R2 are not hydrogen at the same time;
X is chlorine or bromine;
Wherein said lipophilic moiety is selected from the group consisting of C₈₋₂₄ alkyl, monounsaturated, diunsaturated and triunsaturated alkenyl

In a preferred embodiment of the present invention, the disclosed aspartic acid based cationic amphiphile is represented by structure 1 wherein R₁ = R₂ = n-hexadecyl, and X- is a chloride ion.

In another second preferred embodiment of the present invention, the disclosed aspartic acid based cationic I amphiphile is represented by structure 2 wherein R₁ = R₂. = n-octadecyl, and X⁻ is a chloride ion.

In another third preferred embodiment of the present invention, the disclosed glutamic acid based cationic amphiphile is represented by structure 3 wherein R₁ = R₂ = n-hexadecyl, and X- is a chloride ion.

In another fourth preferred embodiment of the present invention, the disclosed glutamic acid based cationic amphiphile is represented by structure 4 wherein R₁ = R₂ = n-octadecyl, and X⁻ is a chloride ion.

The cationic amphiphiles of the present invention have a lipophilic domain that facilitates the formation of lipid complexes or aggregates in aqueous solutions. The lipophilicity of the hydrophobic domains and the hydrophilicity of the polar histidine in head-group domains are such that when the cationic lipids are confronted with aqueous solutions, lipid aggregates are formed in the presence or absence of a second compound. Exemplary lipophilic R₁ and R₂ groups include (1) saturated C₈-C₂₄ alkyl groups and (2) unsaturated C₈-C₂₂ alkenyl groups containing 1, 2, 3 & 4 double bonds. Synthetic strategies employed for preparing the presently described aspartic acid based histidinylated cationic amphiphiles (X) are depicted below schematically in Schemes 1. Aspartic acid based histidinylated cationic amphiphiles (X, Scheme 1) were synthesized by coupling the starting alcohol shown in Scheme 1 with appropriately protected aspartic acid derivative (containing Boc- protected alpha-amine groups). The coupled product (intermediate I, Scheme 1) was deprotected using TFA and the resulting amino compound (intermediate II, Scheme 1) upon peptide coupling with N^{im}, N^{□} di-Boc-L-histidine derivative afforded the intermediate III (Scheme 1). The intermediate III upon conventional acid deprotection followed by chloride ion exchange over Amberlyst A-26 Chloride ion exchange resin afforded the target cationic amphiphiles X (Scheme 1). Details of synthetic procedures for cationic amphiphiles X are described below in Example 1 for synthesis of cationic amphiphile 1 & 2 (as a representative example). Similar synthetic strategies were employed for preparing glutamic acid based histidinylated cationic amphiphiles (Y, Scheme 2) using suitable protected glutamic acid derivative. The details of synthetic procedures for cationic amphiphiles Y are described below in Example 2. Structures of all the synthetic intermediates and target cationic amphiphiles shown in Schemes 1-2 were confirmed by ¹H NMR and ESI mass spectroscopy and the purities (> 95 %) of all the target cationic amphiphiles were confirmed by reverse phase analytical HPLC using pure methanol and 95:5 methanol:water, v/v, as the mobile phase.
HPLC Conditions:
   System: Agilent 1100 series
   Column: Lichrospher® 100, RP-18e (5 µm)
   Mobile Phases: Methanol (A); Methanol:Water, 95:5, v/v, (B).
   Flow Rate: 1.0 mL/min
   Typical Column Pressure: 60-65 Bars
   Detection: UV at 210 nm.

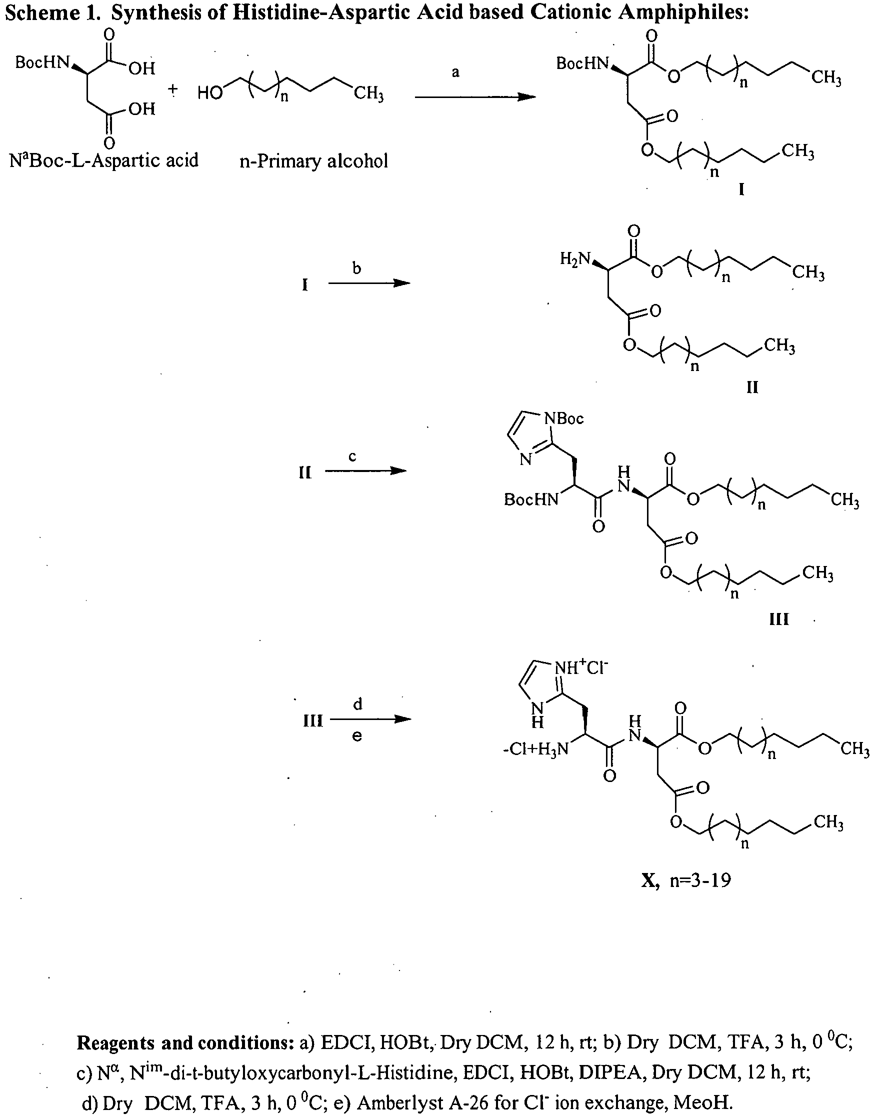

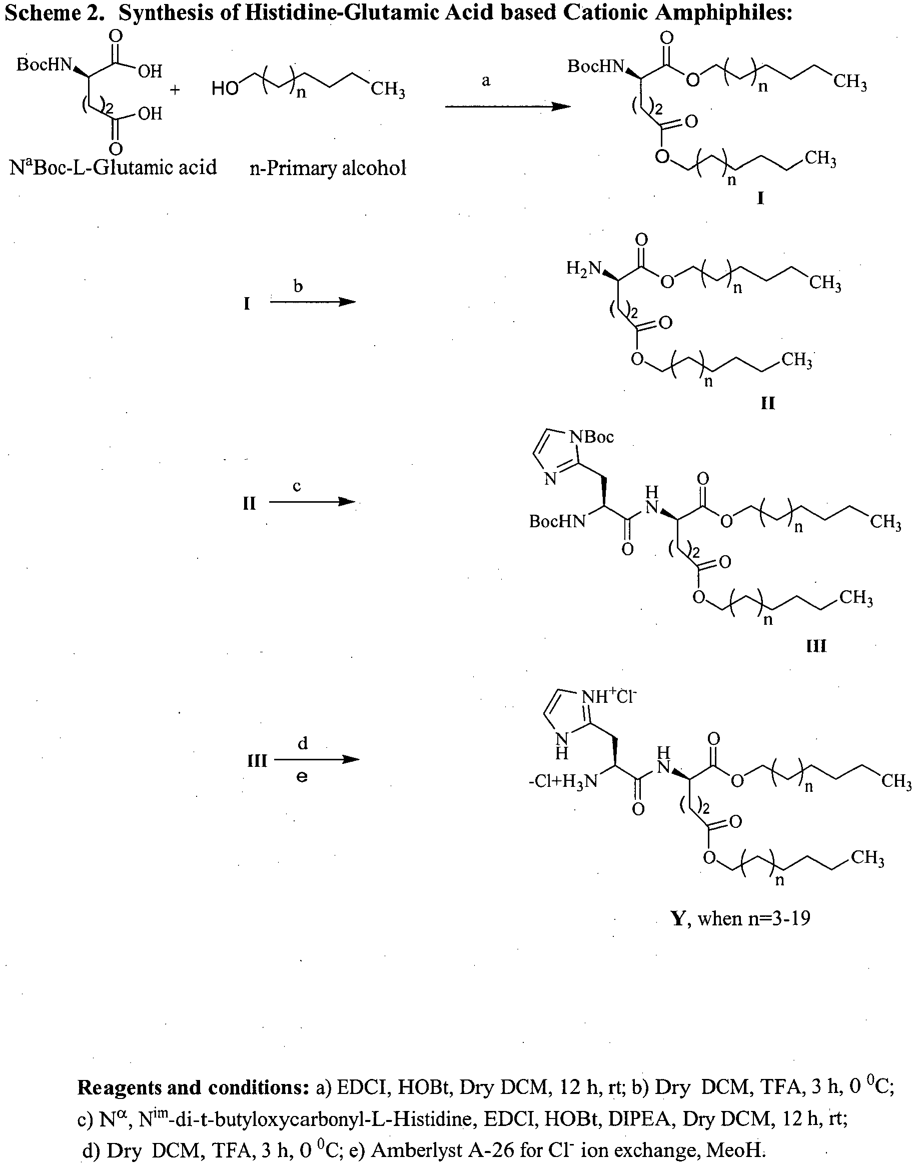

### FORMULATIONS

The present invention also provides novel formulation comprising optimal amounts of cationic amphiphiles as disclosed herein, biological macromolecules and the co-lipids. One or more additional physiologically acceptable substances may be included in the pharmaceutical formulation of the invention to stabilize the formulation for storage or to facilitate successful intracellular delivery of the molecules. Co-lipids according to the practice of the present invention are useful in mixing with one or more of the histidinylaed amphiphiles. DOPC and DOPE are excellent co-lipids for use in combination with the presently described amphiphiles to facilitate successful delivery into cells. DOPC and a helper lipid Q16TG (Figure 1) were used in another combination with the presently described amphiphiles to facilitate successful delivery into cells. A preferred range of molar ratio of the cationic amphiphile to co-lipids DOPC & Q16TG is 1:1:1. As such, it is within the art to vary the said range to a considerably wide extent. Typically, liposomes were prepared by dissolving the cationic amphiphiles and the co-lipid or the helper lipid (DOPC and DOPE or DOPC and Q16TG) in the appropriate mole ratio in a mixture of methanol and chloroform in a glass vial. The solvent was removed with a thin flow of moisture free nitrogen gas and the dried lipid film was then kept under high vacuum for 6-8 h. The dried lipid film was hydrated in sterile deionized water in a total volume of 1 mL at cationic lipid concentration of 1 mM for a minimum of 12 h. Liposomes were vortexed for 1-2 minutes to remove any adhering lipid film and sonicated in a bath sonicator (ULTRAsonik 28X) for 2-3 minutes at room temperature to produce multilamellar vesicles (MLV). MLVs were then sonicated with a Ti-probe (using a Branson 450 sonifier at 100% duty cycle and 25 W output power) for 1-2 minutes to produce small unilamellar vesicles (SUVs) as indicated by the formation of a clear translucent solution. The cationic amphiphiles with histidine head-groups disclosed herein may be blended such that one or more of the representatives thereof may be used in a combination to facilitate entry of the said biologically active molecules into cells/tissues.

In a further embodiment, the cationic amphiphiles disclosed in the present invention may be used in combination with other lipids or helper lipids such as phosphatidylethanolamine, phosphatidylglycerol, etc. The said therapeutic formulation may be stored at 0-4°C. Agents that prevent bacterial growth and increase the shelf life may be included along with reagents that stabilize the preparation, e.g., low concentrations of glycerol. It is specifically warned that freezing and thawing cycles could cause loss in efficiency of the formulation.

In yet another embodiment, the formulation of the cationic amphiphiles disclosed herein, co-lipids (DOPC and DOPE, DOPC and Q16TG) may be administered intravenously besides other routes such as subcutaneous, intramuscular and intraperitonial. Further, the said formulations may be administered to cells at a range of 2-30 micromolar concentration of the histidinylated lipid to 10,000 cells in an in vitro system.

### TOXICITY STUDIES

To discover the anti-cancer property of newly invented histidinylated lipid, first we evaluated the cytotoxic effect of histidinylated lipids in several malignant and non-malignant cell lines using MTT assays. The cytotoxicities of these lipids in this invention were evaluated in A549, B16F10, MCF-7, CHO, C-26, RAW 264.7 & COS-1 cells across the lipid concentration 4-40 micromolar range using MTT based cell viability assay (Figures 2- 8). The yellow tetrazolium salt (MTT) is reduced by mitochondrial dehydrogenase enzyme from metabolically active cells to form insoluble purple formazan crystals, which are solubilized by the addition of a mixture of DMSO & MeOH. The color can then be quantified by spectrophotometric method. For each cell type a linear relationship between cell number and absorbance is established, enabling accurate, straightforward quantification of changes in proliferation. At the highest lipid concentration (40 micromolar), lipids HD-16 (1), HE-16 (3) & HE-18 (4) exhibited nearly 15-30% cell viability after 24 h of treatment. After 24 h, these lipids show 50% cell viability around 15-20 micromolar range in B16F10 cell line. Whereas after 48 h, the effect of all these four lipids are increased (Figure 2A, B). In A549 cell line, at the highest lipid concentration 40 micromolar, lipids HD-16, HD-18, HE-16 & HE-18 exhibited nearly 20-40% cell viability after 24 h. After 24 h, all the lipids show 50% cell viability around 10-17 micromolar range in A549 cell line. After 48 h, all these four lipids show enhanced cytotoxic effect (Figure 3A, B). In MCF-7 cell line, lipids HD-16, HE-16, HD-18 & HE-18 exhibited nearly 20-30% cell viability after 24 h at highest concentration. Whereas at lower concentration, as well as after 48 h HE-16 & HE-18 showed stronger cytotoxic effect than other two (Figure 4A, B). Another important finding of this invention is that all the histidinylated lipids show better cytotoxicity than that of the similar liposomal formulations of the commercially available hydrophobic anti-cancer drug dexamethasone in all three cancerous cells. In CHO cell line, lipids HD-16, HE-16 & HE-18 exhibited most potent cytotoxicity after 24 h as well as after 48 h (Figure 5A-B). Similar trends were observed when C-26 (mouse colon carcinoma, Figure 6A, B) cells were treated with these histidinylated lipids. Lipids HD-16, HE-16, HD-18 & HE-18 exhibited more than 80% cell viability in normal RAW-264.7 cells (Figure 7A-B) & showed ∼70% cell viability in normal COS-1 cells (Figure 8A-B) after 24 and 48 h.

### IN VITRO APOPTOSIS INDUCTION BY HISTIDINYLATED LIPIDS

To quantify apoptotic cell death, we studied the in vitro apoptosis induction efficacy of the presently disclosed histidinylated cationic amphiphiles in mouse melanoma (B16F10) and human lung carcinoma (A549) as model malignant cells by flow cytometry using the FITC-Annexin V Apoptosis Detection Kit (Sigma). This assay provides a simple and effective method to detect apoptosis at a very early stage (Dacharay-Prigent, J. et al. Blood 1993, 81, 2554-2565). It takes advantage of the fact that phosphatidylserine (PS) is translocated from the inner (cytoplasmic) leaflet of the plasma membrane to the outer (cell surface) leaflet soon after the induction of apoptosis and that the annexin V protein has a strong & specific affinity for PS (Zhang, G. et al. BioTechniques 1997, 23, 525-531). PS on the outer leaflet in cells undergoing apoptosis is available for binding to fluorescently labeled annexin V providing the basis for a simple staining assay. The Annexin V assay is a one-step procedure requiring just 10 min to perform. The assay is nonenzymatic, does not require fixation, and is suitable for both adherent and suspension cells. This assay was performed in both B16F10 (Figure 9) as well as A549 cells (Figure 10).

B16F10 & A549 cells were treated with all four histidinylated lipids and liposomal formulation of hydrophobic anti-cancer drug dexamethasone containing DOPC and the helper lipid Q16TG (in 1:1:1 mole ratio of histidinylated lipid:DOPC:Q16TG, 25 µM/mL). Only DOPC and helper lipid Q16TG (containing 1:1 mole ratio) combination was used as control. After 24 h of treatment, cells were stained with annexin V and PI (according to manufacturer protocol, Sigma) and analysed in a flow cytometer (BD FacsCanto II). HD-16 & HE-16 showed enhanced apoptosis in both B16F10 and A549 cells. The percentage of early apoptotic and late apoptotic cells were significantly higher than those for cells treated with liposomal dexamethasone and vehicle only (e.g. liposomes of DOPC & Q16TG only). Among all the amphiphiles, HD-16 & HE-16 were superior to histidinylated cationic amphiphiles with stearyl chains and liposomal formulation of dexamethasone for inducing cellular apoptosis. Importantly, all four hisdinylated lipids (1-4) were found to be 2.7-3.7 times more efficient than the liposomal formulation of the commercially available dexamethasone in inducing apoptosis in B16F10 cells. Similarly, the formulations were 2.2-3.2 times more efficient than the liposomal formulation of the commercially available dexamethasone in inducing apoptosis in A549 cells. Taken together, the findings summarized in Figures 9 and 10 demonstrate that the presently disclosed hisdinylated lipids are capable of inducing apoptosis in cancer cells.

### Histidinylated lipids inhibit Bcl-2 & NF-kB based cell survival pathways

Next, toward obtaining mechanistic insights into the origin of the observed in-vitro apoptosis inducing properties of the presently disclosed histidinylated lipids, we conducted conventional RT-PCR (Reversed transcriptase polymerase chain reaction) assays. Effect of the histidinylated lipids on the mRNA expression of various apoptosis related genes were analyzed by RT-PCR. The human lung carcinoma (A549) cells as a reference, were treated with the liposomal formulations of the histidinylated lipid containing DOPC & helper lipid Q16TG (25 µM/mL) and untreated cells were used as control. After 24 h, the total mRNA was isolated by conventional method using trizol. RT-PCR products were resolved on a 2% agarose gel electrophoresis and visualized with ethidium bromide. Our findings are consistent with the apoptosis inducing abilities of the presently disclosed histidinylated lipids being mediated via inhibition of transcription of the anti-apoptotic Bcl-2 & Bcl-xL genes & via activation of pro-apoptotic BAX & BAK signal transduction pathways. The significantly decreased mRNA levels of Bcl-2 & Bcl-xL were observed when the cells were treated with liposomes of HD-16 & HE-16. However the other two lipids HD-18 & HE-18 did not show significant down regulation of Bcl-2 & Bcl-xL genes. Interestingly, lipids HD-16, HD-18, HE-16 & HE-18 showed up regulation in the expression of pro-apoptotic gene BAX as well as up regulation of Caspase 3 & Caspase 9. 18S m-RNA was used as loading control for all these lanes (Figure 11). Taken together, the findings summarized in Figure 11 demonstrate that several apoptotic signaling pathways get activated upon incubating cancer cells with the liposomal formulations of the presently described histidinylated cationic amphiphiles.

### IN VIVO TUMOUR GROWTH INHIBITION STUDIES

Finally to evaluate therapeutic potentials of the presently disclosed novel histidinylated cationic amphiphiles, we studied the tumor growth inhibition properties of the liposomal formulations of the histidinylated amphiphiles under systemic settings. C57BL/6J mice were used as a reference in-vivo model. Significant tumour growth inhibition by the histidinylated cationic amphiphiles were observed in C57BL/6J mice bearing aggressive B16F10 melanoma tumor. Histidinylated cationic amphiphiles with variable alkyl chain length (HD-16, HD-18, HE-16, HE-18). For comparison sake, liposomal formulation of the commercially available hydrophobic anti-cancer drug dexamethasone (containing 1:1:1 mole ratio of histidinylated lipid:DOPC:Q16TG) were intra-tumorally injected. As depicted in Figure 12 (Parts A & B), significant tumor growth inhibitions were observed with liposomal formulations of the histidinylated cationic amphiphiles and dexamethasone compared to tumor growth inhibition in control untreated mice. However HD-16 & HE-16 showed the highest tumor growth inhibition characteristics; liposomal formuations of HD-18, HE-18 & Dexamethasone showed moderate tumour growth inhibition (Figure 12). Toward confirming that the presently disclosed histidinylated lipids, and not the other liposomal ingredients, play crucial role in mediating observed tumour growth inhibition property, liposomes of only DOPC and helper lipid Q16TG (containing 1:1 mole ratio) combination was used as control. Importantly, such control liposomal formulation containing only Q16TG & DOPC didn't show any tumor growth inhibition effect (Figure 12).

Histidinylated lipids can induce apoptosis in both tumor cells and tumor endothelial cells:
Toward obtaining further mechanistic insights as to whether apoptosis of both tumor and tumor endothelial cells are behind the presently observed tumor growth inhibition, a series of immunohistochemical studies were performed. B16F10 murine melanoma cells were implanted (s.c.) in female C57BL/6J mice and the tumors were allowed to grow and become vascularized for 15 days. At this point, liposomal formulations of the histidinylated cationic amphiphiles as well as the control liposomal formulations with only DOPC and helper lipid Q16TG were intra-tumourally injected into mice containing aggressive B16 tumors (∼2000 mm³) for three consecutive days. Mice were sacrificed 24 h post third day's injection and the tumor was excised, cryosectioned, fixed and the fixed frozen sections were stained with Promega TUNEL Assay kit according to manufacturer's protocol for marking apoptotic cells. Subsequently, the same tumor cryosections were immunounostained with anti-VE-cadherin monoclonal antibody (endothelial cell marker, Satna Cruz) followed by texas-red attached secondary antibody treatment to identify tumor vasculatures. Significant amount of both green (TUNEL positive) and red (endothelial cell positive) fluorescence were visualized under a fluorescence microscope (Figure 13, Parts A & B respectively). Interestingly the merged panel C (mixing of Part A & B) in figure 13 shows presence of yellow, green and red colours. Presence of yellow colour (due to mixing of green and red colors) confirmed apoptosis in tumour endothelial cells. Abundance of some free green fluorescently labeled cells confirmed simultaneous apoptosis of tumour cells. These findings are consistence with the notion that the liposomes of the presently disclosed histidinylated cationic amphiphiles are capable of inducing apoptosis in both tumor cells and tumor endothelial cells.

### APPLICATIONS

The process of the present invention can be exploited for preparing aspartic and glutamic acid based cationic amphiphiles containing histidine in head-group region. The invention provides liposomal compositions of novel histidinylated lipids containing the said cationic amphiphiles with various co-lipids & helper lipid. The liposomal formulations of the cationic amphiphiles described herein are capable to prevent malignant progression and are useful in methods of treatment of cancer in human and animal body. The cationic amphiphiles disclosed herein show enhanced cellular apoptosis through the several apoptotic signal transduction pathways. The presently disclosed compounds are useful in treating various types of cancers including lung, melanoma, breast, colon, ovarian cancers, etc. The compounds also can be used in combination therapy with various other anti-cancer drugs or genes. The presently described novel histidinylated lipids can inhibit the tumor growth in animal body administered via sub-cutaneous, intradermal, intravenous, intramuscular, intraperitonial routes and also capable to induce apoptosis in both tumor endothelial cell and tumor cells. The compounds can inhibit angiogenesis by killing the tumor endothelial cells in tumor micro-environment. Importantly, the liposomal formulations of the presently disclosed histidinylated cationic amphiphiles are more efficient than the similar liposomal composition of commercially available hydrophobic anti-cancer drug dexamethasone in inducing apoptosis of tumor cells under both in vitro and in vivo settings. The area of medical science that is likely to benefit most from the present invention is cancer therapy.

The following examples are given by way of illustration of the present invention and therefore should not be construed to limit the scope of the present invention.

### EXAMPLE 1

### Synthesis of HD-16 (2-((S)-2-ammonio-3-((R)-1,4-bis(hexadecyloxy)-1,4-dioxobutan-2-ylamino)-3-oxopropyl)-1H-imidazol-3-iumchloride) (Scheme 1):

Step (a): Solid HOBt (0.58 g, 3.77 mmol), DIMAP (0.096 g, 0.7 mmol) and EDCl (0.72 g, 3.77 mmol) were added sequentially to an ice cold and stirred solution of N^{α}BOC-L-Aspartic acid (0.40 g, 1.72 mmol) in dry DCM (15 mL). After half an hour, n-hexadecylalcohol (0.80 g, 3.77 mmol) dissolved in dry DCM (10 mL) were added to the reaction mixture. The resulting solution was left stirred at room temperature for 12 h. The solution was diluted in chloroform (80 mL) and washed sequentially with ice-cooled 1N HCl (2 x 80 mL), saturated sodium bicarbonate (2 x 90 mL) and brine (1 x 60 mL). The organic layer was dried over anhydrous sodium sulfate, filtered and the solvent from the filtrate removed by rotary evaporation. The residue upon column chromatographic purification with 60-120 mesh silica gel using 4% ethylacetate-hexane (v/v) as eluent afforded 0.70 g (63% yields) of the pure intermediate I. (R_{f} = 0.8 using 5% chloroform-methanol v/v, as the TLC developing solvent).
   ¹H NMR (200 MHz, CDCl₃):δ/ppm = 0.9 [m, 6H, CH₃-(CH₂)₁₅-]; 1.1-1.6 [bs, 52H,-(CH₂)₁₃-; m, 4H, -(CH₂)₁₃-CH₂-CH₂-O-s, 9H, CO-O-C(CH₃)₃]; 2.4-2.8[m, 2H, Asp C^{β}H₂]; 3.2-3.3[m, 4H, t, 4H, -(CH₂)₁₄-CH₂-O-]; 4.3[m, 1 H, AspC^{α}H]; 6.1-6.3[m, 1 H, NH-CO-O-(CH₃)₃];
   ESIMS : m/z= 681 [M+1]⁺ for C₄₁H₇₉NO₆
Step (b The intermediate **I** (0.55 g, 0.88 mmol) prepared above in step a was dissolved in dry DCM (8 mL) and TFA (4 mL) was added at 0°C. The resulting solution was left stirred at 0°C for 3 h to ensure complete deprotection. Excess TFA was removed by nitrogen flushing. The resulting compound was dissolved in chloroform (80 mL) and washed with aqueous saturated NaHCO₃ (3 x 90 mL), brine (1 x 70 mL). The organic layer was dried over anhydrous sodium sulfate, filtered and the solvent from the.filtrate removed by rotary evaporation afforded 0.41 g (91% yields) of free amine as intermediate **II.** (R_{f} = 0.2 using 10% Methanol-chloroform v/v, as the TLC developing solvent).
Step (c): Solid HoBt (0.15 g, 1.00 mmol) and EDCI (0.19 g, 1.00 mmol) were added sequentially to an ice cold and stirred solution of N^{α}, N^{ω}-di-t-butyloxycarbonyl-L-Histidine (0.36 g, 1.00 mmol) in dry DCM (15 mL). After half an hour, the intermediate II (0.420 g, 0.810 mmol) obtained in step b dissolved in dry DCM (10 mL) were added to the reaction mixture. Di-isopropylethyl amine (DIPEA) was added dropwise to the stirred reaction mixture until it became alkaline to litmus. The resulting solution was left stirred at room temperature for 12 h. The solution was diluted in chloroform (100 mL) and washed sequentially with ice-cooled 1 N HCl (2 x 80 mL), saturated sodium bicarbonate (2 x 70 mL) and brine (1 x 80 mL). The organic layer was dried over anhydrous sodium sulfate, filtered and the solvent from the filtrate removed by rotary evaporation. The residue upon column chromatographic purification with 60-120 mesh silica gel using 1.5 % chloroform-methanol (v/v) as eluent afforded 0.38 g (55% yields) of the pure intermediate **III.** (R_{f} = 0.5 using 5% chloroform-methanol v/v, as the TLC developing solvent).
   ¹H NMR (200 MHz, CDCl₃):δ/ppm = 0.8 [m, 6H, CH₃(CH₂)₁₅-]; 1.0-1.6 [bs, 52H, - (CH₂)13-; s, 9H, CO-O-C(CH₃)₃; m, 4H, -(CH₂)₁₃-CH₂-CH₂-O-; s, 9H, CO-O-C(CH₃)₃]; 2.3-2.9[m, 2H, Asp C^{β}H₂]; 3.0-3.2[m, 4H, t, 4H, -(CH2)₁₄-CH₂-O-; m, 2H, HisC^{β}H ]; 4.2-4.6[m, 1H, AspC^{α}H; m, 1H, HisC^{α}H ]; 6.0[m, 1H, NH-CO-O-(CH₃)₃]; 6.5 [m, 1 H, AspC^{α}H-NH-CO]; 7.7-8.4[ m, 2H, His-ring]
   ESIMS : m/z= 919 [M+1]⁺for C₅₂H₉₆N₄O₉
Step (d): To the ice cold solution of the intermediate **III** (0.2 g, 0.02 mmol) prepared above in step c was dissolved in 2 mL dry DCM. 1 mL of TFA was added and the mixture was allowed to stir for 3-4 h. TFA was removed with nitrogen flow and the residue was subjected to chloride ion exchange chromatography over amberlyst A-26 chloride ion exchange resin. The compound obtained after chloride ion exchange upon recrystalization from 1:5 (v/v) MeOH:Acetone afforded 0.094 g (47% yields) of the pure target compound **HD-16** as a white solid. (R_{f} = ∼0.3, 10% methanol-chloroform, v/v).
   ¹H NMR (200 MHz, CDCl₃+CD₃OD):δ/ppm = 0.8 [m, 6H, CH₃-(CH₂)₁₅-]; 1.0-1.6 [bs, 52H, -(CH₂)₁₃-; m, 4H, -(CH₂)₁₃-CH₂-CH₂-O]; 2.2 [m, 1H, Asp C^{β}H ]; 2.6[m, 1H, Asp C^{β}H]; 3.0-4.6[m, 4H, t, 4H, -(CH₂)₁₃-CH₂-CH₂-O-; m, 2H, HisC^{β}H; m, 1 H, AspC^{α}H; m, 1H, HisC^{α}H + CD₃OH+ CH₃OD ]; 8.5-8.6[m, 2H, His-ring]
   ESIMS : m/z= 719 [M+1]⁺ for C₄₂H₈₀N₄O₅

### EXAMPLE 2

### Synthesis of HD-18 (2-((S)-2-ammonio-3-((R)-1,4-bis(octadecyloxy)-1,4-dioxobutan-2-ylamino)-3-oxopropyl)-1H-imidazol-3-iumchloride) (Scheme 1):

Step (a): Solid HOBt (0.58 g, 3.77 mmol), DIMAP (0.096 g, 0.7 mmol) and EDCl (0.72 g, 3.77 mmol) were added sequentially to an ice cold and stirred solution of N^{α}BOC-L-Aspartic acid (0.40 g, 1.72 mmol) in dry DCM (15 mL). After half an hour, n-octadecylalcohol (0.80 g, 3.77 mmol) dissolved in dry DCM (10 mL) were added to the reaction mixture. The resulting solution was left stirred at room temperature for 12 h. The solution was diluted in chloroform (80 mL) and washed sequentially with ice-cooled 1N HCl (2 x 80 mL), saturated sodium bicarbonate (2 x 90 mL) and brine (1 x 60 mL). The organic layer was dried over anhydrous sodium sulfate, filtered and the solvent from the filtrate removed by rotary evaporation. The residue upon column chromatographic purification with 60-120 mesh silica gel using 4% ethylacetate-hexane (v/v) as eluent afforded 0.70 g (63% yields) of the pure intermediate I. (R_{f} = 0.8 using 5% chloroform-methanol v/v, as the TLC developing solvent).
   ¹H NMR (200 MHz, CDCl₃):δ/ppm = 0.9 [m, 6H, CH₃-(CH₂)₁₇-]; 1.1-1.6 [bs, 60H,-(CH₂)₁₅-; m, 4H, -(CH₂)₁₅-CH₂-CH₂-O-_{S}, 9H, CO-O-C(CH₃)₃]; 2.4-2.8[m, 2H, Asp C^{β}H₂ ]; 3.2-3.3[m, 4H, t, 4H, -(CH₂)₁₆-CH₂-O-]; 4.3[m, 1H, AspC^{α}H ]; 6.1-6.3[m, 1H, NH-CO-O-(CH₃)₃];
   ESIMS : m/z= 737 [M+1]⁺ for C₄₅H₈₇NO₆
Step (b The intermediate **I** (0.55 g, 0.88 mmol) prepared above in step a was dissolved in dry DCM (8 mL) and TFA (4 mL) was added at 0°C. The resulting solution was left stirred at 0°C for 3 h to ensure complete deprotection. Excess TFA was removed by nitrogen flushing. The resulting compound was dissolved in chloroform (80 mL) and washed with aqueous saturated NaHCO₃ (3 x 90 mL), brine (1 x 70 mL). The organic layer was dried over anhydrous sodium sulfate, filtered and the solvent from the filtrate removed by rotary evaporation afforded 0.41 g (91% yields) of free amine as intermediate **II**. (R_{f} = 0.2 using 10% Methanol-chloroform v/v, as the TLC developing solvent).
Step (c): Solid HoBt (0.15 g, 1.00 mmol) and EDCl (0.19 g, 1.00 mmol) were added sequentially to an ice cold and stirred solution of N^{α}, N^{ω}-di-t-butyloxycarbonyl-L-Histidine (0.36 g, 1.00 mmol) in dry DCM (15 mL). After half an hour, the intermediate **II** (0.420 g, 0.810 mmol) obtained in step b dissolved in dry DCM (10 mL) were added to the reaction mixture. Di-isopropylethyl amine (DIPEA) was added dropwise to the stirred reaction mixture until it became alkaline to litmus. The resulting solution was left stirred at room temperature for 12 h. The solution was diluted in chloroform (100 mL) and washed sequentially with ice-cooled 1N HCl (2 x 80 mL), saturated sodium bicarbonate (2 x 70 mL) and brine (1 x 80 mL). The organic layer was dried over anhydrous sodium sulfate, filtered and the solvent from the filtrate removed by rotary evaporation. The residue upon column chromatographic purification with 60-120 mesh silica gel using 1.5 % chloroform-methanol (v/v) as eluent afforded 0.38 g (55% yields) of the pure intermediate **III.** (R_{f} = 0.5 using 5% chloroform-methanol v/v, as the TLC developing solvent).
   ¹H NMR (200 MHz, CDCl₃):δ/ppm = 0.8 [m, 6H, CH₃-(CH₂)₁₇-]; 1.0-1.6 [bs, 52H, - (CH₂)₁₅-; s, 9H, CO-O-C(CH₃)₃; m, 4H, -(CH₂)₁₅-CH₂-CH₂-O-; s, 9H, CO-O-C(CH₃)₃]; 2.3-2.9[m, 2H, Asp C^{β}H₂]; 3.0-3.2[m, 4H, t, 4H, -(CH₂)₁₆-CH₂-O-; m, 2H, HisC^{β}H]; 4.2-4.6[m, 1H, AspC^{α}H; m, 1H, HisC^{α}H ]; 6.0[m, 1H, NH-CO-O-(CH₃)₃]; 6.5 [m, 1H, AspC^{α}H-NH-CO]; 7.7-8.4[ m, 2H, His-ring]
   ESIMS : m/z= 975 [M+1]⁺ for C₅₆H₁₀₄N₄O₉
Step (d): To the ice cold solution of the intermediate **III** (0.2 g, 0.02 mmol) prepared above in step c was dissolved in 2 mL dry DCM. 1 mL of TFA was added and the mixture was allowed to stir for 3-4 h. TFA was removed with nitrogen flow and the residue was subjected to chloride ion exchange chromatography over amberlyst A-26 chloride ion exchange resin. The compound obtained after chloride ion exchange upon recrystalization from 1:5 (v/v) MeOH:Acetone afforded 0.094 g (47% yields) of the pure target compound **HD**-**18** as a white solid. (R_{f} = ∼0.3,10% methanol-chloroform, v/v).
   ¹H NMR (200 MHz, CDCl₃+CD₃OD):δ/ppm = 0.8 [m, 6H, CH₃-(CH₂)₁₇-]; 1.0-1.6 [bs, 52H, -(CH₂)₁₅-; m, 4H, -(CH₂)₁₅-CH₂-CH₂-O]; 2.2 [m, 1H, Asp C^{β}H ]; 2.6[m, 1H, Asp C^{β}H]; 3.0-4.6[m, 4H, t, 4H, -(CH₂)₁₅-CH₂-CH₂-O-; m, 2H, HisC^{β}H; m, 1H, AspC^{α}H; m, 1 H, HisC^{α}H + CD₃OH+ CH₃OD ]; 8.5-8.6[m, 2H, His-ring]
   ESIMS : m/z= 775 [M+1]⁺for C₄₂H₈₀N₄O₅

### EXAMPLE 3

### Synthesis of HE-16 (2-((S)-2-ammonio-3-((R)-1,5-bis(hexadecyloxy)-1,5-dioxopentan-2-ylamino)-3-oxopropyl)-1H-imidazol-3-iumchloride) (Scheme 2):

Step (a): Solid HOBt (0.76 g, 4.96 mmol), EDCI (0.94 g, 4.96 mmol) and DIMAP (0.096 g, 0.7 mmol) were added sequentially to an ice cold and stirred solution of NαBOC-L-glutamic acid (0.50 g, 1.9 mmol) in dry DCM (15 mL). After half an hour, n-hexadecylalcohol (1.19 g, 4.96 mmol) dissolved in dry DCM (20 mL) was added to the reaction mixture. The resulting solution was left stirred at room temperature for 12 h. The solution was diluted in chloroform (80 mL) and washed sequentially with ice-cooled 1N HCl (2 x 80 mL), saturated sodium bicarbonate (2 x 90 mL) and brine (1 x 60 mL). The organic layer was dried over anhydrous sodium sulfate, filtered and the solvent from the filtrate removed by rotary evaporation. The residue upon column chromatographic purification with 60-120 mesh silica gel using 4% ethylacetate-hexane (v/v) as eluent afforded 1.20 g (73%, yields) of the pure intermediate **I.** (Rf = 0.8 using 5% chloroform-methanol v/v, as the TLC developing solvent).
   Intermediate **I, HE-16:**
   ¹H NMR (300 MHz, CDCl₃):δ/ppm = 0.9 [m, 6H, CH₃-(CH₂)₁₅-]; 1.1-1.6 [bs, 44H, - (CH₂)₁₃-; m, 4H, -(CH₂)₁₃-CH₂-CH₂-O-s, 9H, CO-O-C(CH₃)₃]; 2.4-2.8[m, 4H, Glu C^{β,γ}H₂ ]; 3.2-3.3[m, 4H, t, 4H, -(CH₂)₁₄-CH₂-O-]; 4.3[m, 1H, GluC^{α}H, m, 2H, CH₃-(CH₂)₁₃-0-CO-C^{α}H].
   ESIMS : m/z= 695 [M+1]+ for C₄₂H₈₁NO₆
Step (b): The intermediate I (0.5 g, 0.8 mmol) prepared in above step a, was dissolved in dry DCM (4 mL) and TFA (2 mL) was added at 0°C. The resulting solution was left stirred at 0°C for 3 h to ensure complete deprotection. Excess TFA was removed by nitrogen flushing. The resulting compound was dissolved in chloroform (80 mL) and washed with aqueous saturated NaHCO₃ (3 x 90 mL), brine (1 x 70 mL). The organic layer was dried over anhydrous sodium sulfate, filtered and the solvent from the filtrate removed by rotary evaporation afforded 0.41 g (91% yield) of free amine as intermediate II.
Step (c): Solid HoBt (0.15 g, 1.00 mmol) and EDCI (0.19 g, 1.00 mmol) were added sequentially to an ice cold and stirred solution of Nα, Nω-di-t-butyloxycarbonyl-L-Histidine (0.28 g, 0.79 mmol) in dry DCM (15 mL). After half an hour, the intermediate II (0.410 g, 0.660 mmol) obtained in step b dissolved in dry DCM (10 mL) were added to the reaction mixture. Di-isopropylethyl amine (DIPEA) was added dropwise to the stirred reaction mixture until it became alkaline to litmus. The resulting solution was left stirred at room temperature for 12 h. The solution was diluted in chloroform (100 mL) and washed sequentially with ice-cooled 1 N HCl (2 x 80 mL), saturated sodium bicarbonate (2 x 70 mL) and brine (1 x 80 mL). The organic layer was dried over anhydrous sodium sulfate, filtered and the solvent from the filtrate removed by rotary evaporation. The residue upon column chromatographic purification with 60-120 mesh silica gel using 1.5 % methanol-chloroform (v/v) as eluent afforded 0.38 g (55%) of the pure intermediate III. (Rf = 0.5 using 5% chloroform-methanol v/v, as the TLC developing solvent).
   Intermediate III, **HE-16:**
   ¹H NMR: (300 MHz, CDCl₃):δ/ppm = 0.9 [m, 6H, CH₃-(CH₂)₁₅-]; 1.1-1.6 [bs, 44H, - (CH₂)₁₃-; m, 4H, -(CH₂)₁₃-CH₂-CH₂-O-s, 18H, CO-O-C(CH₃)₃]; 2.4-2.8[m, 4H, Glu C^{β,γ}H₂ ]; 3.0-3.2[m, 4H, t, 4H, -(CH₂)₁₂-CH₂-O-; m, 2H, HisC^{β}H]; 3.2-3.3[m, 4H, t, 4H, - (CH₂)₁₄-CH₂-O-]; 4.2-4.4[m, 1 H, GluC^{α}H; m, 1 H, HisC^{α}H ; m,] 7.2-8.4[ m, 2H, His-ring] ESIMS : m/z= 934 [M+1]⁺ for C₅₃H₉₅N₄O₉
Step (d): To the ice cold solution of the intermediate **III** (0.2 g, 0.02 mmol) prepared above in step c was dissolved in 2 mL dry DCM. 1 mL of TFA was added and the mixture was allowed to stir for 3-4 h. TFA was removed with nitrogen flow and the residue was subjected to chloride ion exchange chromatography over amberlyst A-26 chloride ion exchange resin. The compound obtained after chloride ion exchange upon recrystalization from 1:5 (v/v) MeOH:Acetone afforded 0.094 g (47% yields) of the pure target compound **HE-16** as a white solid. (R_{f} = ∼0.3, 10% methanol-chloroform, v/v).
   **HE-16:**
   ¹H NMR (500 MHz, CDCl₃+CD₃OD):δ/ppm = 0.8 [m, 6H, CH₃-(CH₂)₁₅-]; 1.0-1.6 [bs, 44H, -(CH₂)₁₃-; m, 4H, -(CH₂)₁₃-CH₂-CH₂-O]; 2.2 [m, 1H, Glu C^{β}H ]; 2.6[m, 1H, Glu C^{β}H]; 3.0-4.6[m, 4H, t, 4H, -(CH₂)₁₃-CH₂-CH₂-O-; m, 2H, HisC^{β}H; m, 1H, GluC^{α}H; m, 1 H, HisC^{α}H + CD₃OH+ CH₃OD]; 7.5[m, 2H, His-ring]
   ESIMS : m/z= 732 [M+1]⁺ for C₄₃H₈₂N4O₅

### EXAMPLE 4

### Synthesis of HE-18 (2-((S)-2-ammonio-3-((R)-1,5-bis(octaadecyloxy)-1,5-dioxopentan-2-ylamino)-3-oxopropyl)-1 H-imidazol-3-iumchloride) (Scheme 2):

Step (a): Solid HOBt (0.76 g, 4.96 mmol), EDCl (0.94 g, 4.96 mmol) and DIMAP (0.096 g, 0.7 mmol) were added sequentially to an ice cold and stirred solution of NαBOC-L-glutamic acid (0.50 g, 1.9 mmol) in dry DCM (15 mL). After half an hour, n-octadecylalcohol (1.19 g, 4.96 mmol) dissolved in dry DCM (20 mL) was added to the reaction mixture. The resulting solution was left stirred at room temperature for 12 h. The solution was diluted in chloroform (80 mL) and washed sequentially with ice-cooled 1 N HCl (2 x 80 mL), saturated sodium bicarbonate (2 x 90 mL) and brine (1 x 60 mL). The organic layer was dried over anhydrous sodium sulfate, filtered and the solvent from the filtrate removed by rotary evaporation. The residue upon column chromatographic purification with 60-120 mesh silica gel using 4% ethylacetate-hexane (v/v) as eluent afforded 1.20 g (73%, yields) of the pure intermediate I. (Rf = 0.8 using 5% chloroform-methanol v/v, as the TLC developing solvent).
   ¹H NMR (300 MHz, CDCl₃):δ/ppm = 0.9 [m, 6H, CH₃-(CH₂)₁₇-]; 1.1-1.6 [bs, 44H, - (CH₂)₁₃-; m, 4H, -(CH₂)₁₅-CH₂-CH₂-O-s, 9H, CO-O-C(CH₃)₃]; 2.4-2.8[m, 4H, Glu C^{β,γ}H₂ ]; 3.2-3.3[m, 4H, t, 4H, -(CH₂)₁₄-CH₂-O-]; 4.3[m, 1 H, GluC^{α}H, m, 2H, CH₃-(CH₂)₁₃-NH-CO-C^{α}H].
   ESIMS : m/z= 751 [M+1]⁺ for C₄₆H₈₉NO₆
Step (b): The intermediate I (0.5 g, 0.8 mmol) prepared in above step a, was dissolved in dry DCM (4 mL) and TFA (2 mL) was added at 0°C. The resulting solution was left stirred at 0°C for 3 h to ensure complete deprotection. Excess TFA was removed by nitrogen flushing. The resulting compound was dissolved in chloroform (80 mL) and washed with aqueous saturated NaHCO₃ (3 x 90 mL), brine (1 x 70 mL). The organic layer was dried over anhydrous sodium sulfate, filtered and the solvent from the filtrate removed by rotary evaporation afforded 0.41 g (91% yield) of free amine as intermediate **II.**
Step (c): Solid HoBt (0.15 g, 1.00 mmol) and EDCl (0.19 g, 1.00 mmol) were added sequentially to an ice cold and stirred solution of Nα, Nω-di-t-butyloxycarbonyl-L-Histidine (0.28 g, 0.79 mmol) in dry DCM (15 mL). After half an hour, the intermediate **II** (0.410 g, 0.660 mmol) obtained in step b dissolved in dry DCM (10 mL)were added to the reaction mixture. Di-isopropylethyl amine (DIPEA) was added dropwise to the stirred reaction mixture until it became alkaline to litmus. The resulting solution was left stirred at room temperature for 12 h. The solution was diluted in chloroform (100 mL) and washed sequentially with ice-cooled 1N HCl (2 x 80 mL), saturated sodium bicarbonate (2 x 70 mL) and brine (1 x 80 mL). The organic layer was dried over anhydrous sodium sulfate, filtered and the solvent from the filtrate removed by rotary evaporation. The residue upon column chromatographic purification with 60-120 mesh silica gel using 1.5 % methanol-chloroform (v/v) as eluent afforded 0.38 g (55%) of the pure intermediate **III.** (Rf = 0.5 using 5% chloroform-methanol v/v, as the TLC developing solvent).
   ¹H NMR (300 MHz, CDCl₃):δ/ppm = 0.9 [m, 6H, CH₃-(CH₂)₁₇-]; 1.1-1.6 [bs, 44H,-(CH₂)₁₅-; m, 4H, -(CH₂)₁₅-CH₂-CH₂-O-_{S}, 18H, CO-O-C(CH₃)₃]; 3-3.3[m, 4H, Glu C^{β,γ}H₂]; 3.3-3.5[m, 4H, t, 4H, -(CH₂)₁₆-CH₂-O-; m, 2H, HisC^{β}H]; 3.5-4[m, 4H, t, 4H, -(CH₂)₁₄-CH₂-O-]; 4.2-4.4[m, 1H, GluC^{α}H; m, 1H, HisC^{α}H ; m,] 7-8[ m, 2H, His-ring]
   ESIMS : m/z= 989 [M+1]⁺ for C₅₇H₁₀₃N₄O₉
Step (d): To the ice cold solution of the intermediate **III** (0.2 g, 0.02 mmol) prepared above in step c was dissolved in 2 mL dry DCM. 1 mL of TFA was added and the mixture was allowed to stir for 3-4 h. TFA was removed with nitrogen flow and the residue was subjected to chloride ion exchange chromatography over amberlyst A-26 chloride ion exchange resin. The compound obtained after chloride ion exchange upon recrystalization from 1:5 (v/v) MeOH:Acetone afforded 0.094 g (47% yields) of the pure target compound **HE-18** as a white solid. (R_{f} = ∼0.3, 10% methanol-chloroform, v/v).
   ¹H NMR (500 MHz, CDCl₃+CD₃OD):δ/ppm = 0.8 [m, 6H, CH₃-(CH₂)₁₇-]; 1.0-2.7 [bs, 44H, -(CH₂)₁₅-; m, 4H, -(CH₂)₁₅-CH₂-CH₂-O; m, 1 H, Glu C^{β}H, m, 1 H, Glu C^{β}H]; 2.8-4.6[m, 4H, t, 4H, -(CH₂)₁₅-CH₂-CH₂-O-; m, 2H, HisC^{β}H; m, 1H, GluC^{α}H; m, 1 H, HisC^{α}H + CD₃OH+ CH₃OD ]; 8-8.3[m, 2H, His-ring]
   ESIMS : m/z= 790 [M+2]⁺ for C₄₇H₉₀N₄O₅

### EXAMPLE 5

Preparation of liposomes. Histidinylated cationic lipids (HD-16, HD-18, HE-16 & HE-18), co-lipids (DOPE, DOPC) and helper lipid Q16TG ranging from 1:1:1 to 3:2:1 molar ratios were dissolved in chloroform. The solvent was then evaporated under a thin stream of nitrogen gas, vacuum dried for 8 h and hydrated in deionised water overnight to give a final lipid concentration of 1 mM for in vitro experiments or 5 mM for in vivo experiments. The hydrated lipid film was first vortexed for 30 seconds and then sonicated until clarity using a Branson 450 sonifier at 100% duty cycle and 25 W output power. The resulting clear aqueous liposomes were used for treatment.

### EXAMPLE 6

**MTT Assay.** Cells were seeded at a density of 10000 (for B16F10, A549,MCF-7 & CHO) 0r 15000(for Raw264.7) per well in a 96-well plate for 18-24 h before the treatment. Cells were incubated with the liposomes of the presently described histidinylated cationic amphiphiles in the concentration range 40-16 µmol/mL in serum free medium. Immediately before the treatment, cells plated in the 96-well plate were washed with PBS (2 x 100 µL). After 4 h of incubation, the medium was replaced with fresh complete medium containing 10% FBS. The 10 µl of freshly preapared MTT solution in PBS (5 mg/mL) was added to each well of the plate after 24 or 48 h. The yellow tetrazolium salt (MTT) was reduced by mitochondrial dehydrogenase enzyme from metabolically active cells to form insoluble purple formazan crystals, which are solubilized by the addition of a mixture of DMSO & MeOH. The intensities of color were then quantified by spectrophotometric means. For each cell type a linear relationship between cell number and absorbance was established, enabling accurate, straightforward quantification of changes in proliferation.

Results: At the highest lipid concentration (40 micromolar), lipids HD-**16** (**1**), HE-**16** (**3**) & HE**-18** (**4**) exhibited nearly 15-30% cell viability after 24 h of treatment. After 24 h, these lipids show 50% cell viability around 15-20 micromolar range in B16F10 cell line. Whereas after 48 h, the effect of all these four lipids are increased (Figure 2A, B). In A549 cell line, at the highest lipid concentration 40 micromolar, lipids **HD-16**, **HD-18, HE-16 & HE-18** exhibited nearly 20-40% cell viability after 24 h. After 24 h, all the lipids show 50% cell viability around 10-17 micromolar range in A549 cell line. After 48 h, all these four lipids show enhanced cytotoxic effect (Figure 3A, B). In MCF-7 cell line, lipids **HD-16, HE-16, HD-18 & HE-18** exhibited nearly 20-30% cell viability after 24 h at highest concentration. Whereas at lower concentration, as well as after 48 h HE-**16** & HE-**18** showed stronger cytotoxic effect than other two (Figure 4A, B). Another important finding of this invention is that all the histidinylated lipids show better cytotoxicity than that of the similar liposomal formulations of the commercially available hydrophobic anti-cancer drug dexamethasone in all three cancerous cells. In CHO cell line, lipids **HD-16, HE-16 & HE-18** exhibited most potent cytotoxicity after 24 h as well as after 48 h (Figure 5A-B). Similar trends were observed when C-26 (mouse colon carcinoma, Figure 6A, B) cells were treated with these histidinylated lipids. Lipids **HD-16, HE-16, HD-18 & HE-18** exhibited more than 80% cell viability in normal RAW-264.7 cells (Figure 7A-B) & showed ∼70% cell viability in normal COS-1 cells (Figure 8A-B) after 24 and 48 h.

### EXAMPLE 7

**Flow Cytometry:** A459 as well as B16f10 cells were seeded at a density of 10⁶ cells/well in a 6 well plate usually 18-24 h before treatment. 30 µL liposomes of the presently described histidinylated cationic amphiphiles (1 mmol/mL) diluted to 1 mL with plain DMEM and the diluted solutions were gently shaken for 5 min. Cells were washed with phosphate-buffered saline (PBS), pH 7.4 (1 x 100 µL) and the 1 mL liposomal solutions were added to the cells. After 4 h of incubation, the medium was replaced with fresh complete medium containing 10% FBS. Then the cells were incubated at a humidified atmosphere containing 5% CO₂ at 37 °C for 24 h. After 24 h of incubation, cells were trypsinized, centrifuged and the pellets were resuspended in 500 µL binding buffer containing 5 µL of annexin-V FITC and 5 µL of PI. The mixture was then incubated for 20 min at dark (BD, USA) and analysis was immediately performed using a flow cytometer (FACS Canto II, BD).

Results: HD-**16** & HE-**16** showed enhanced apoptosis in both B16F10 and A549 cells. The percentage of early apoptotic and late apoptotic cells were significantly higher than those for cells treated with liposomal dexamethasone and vehicle only (e.g. liposomes of DOPC & Q16TG only). Among all the amphiphiles, HD-**16** & HE-**16** were superior to histidinylated cationic amphiphiles with stearyl chains and liposomal formulation of dexamethasone for inducing cellular apoptosis. Importantly, all four hisdinylated lipids (**1-4**) were found to be 2.7-3.7 times more efficient than the liposomal formulation of the commercially available dexamethasone in inducing apoptosis in B16F10 cells. Similarly, the formulations were 2.2-3.2 times more efficient than the liposomal formulation of the commercially available dexamethasone in inducing apoptosis in A549 cells. Taken together, the findings summarized in Figures 9 and 10 demonstrate that the presently disclosed hisdinylated lipids are capable of inducing apoptosis in cancer cells.

### EXAMPLE 8

### RT-PCR (reverse transcriptase-PCR) analysis.

Semi-quantitative RT-PCR analysis was performed to measure the changes in gene expression of treated cells in contrast to the untreated cells. A549 cells (ATCC) were seeded at a density of 10⁶ cells/well in a 6 well plate usually 18-24 h before transfection. 30 µL liposomes of the presently described histidinylated cationic amphiphiles (1 mmol/mL) diluted to 1 mL with plain DMEM and the diluted solutions were gently shaken for 5 min. Cells were washed with phosphate-buffered saline (PBS), pH 7.4 (1 x 100 µL) and the 1 mL liposomal solutions were added to the cells. Cells were washed with phosphate-buffered saline (PBS), pH 7.4 (1 x 100 µL) and the 1 mL liposomal solutions were added to the cells (1 mL). After 4 h of incubation, the medium was replaced with fresh complete medium containing 10% FBS. Then the cells were incubated at a humidified atmosphere containing 5% CO₂ at 37°C for 24 h. After 24 h of transfection, lipoplex treated and untreated cells were lysed directly in 6-well plates with 1 mL of TRIzol® reagent (Invitrogen) following removal of media and washing with PBS (1 mL). The cells were dissolved in TRI Reagent and cell lysates were pipetted several times to clear the haziness. Cell lysates were taken in 1.5 mL centrifuge tubes, after 10 min 0.2 mL chloroform was added. The samples were covered tightly and shaken vigorously for 15 sec, incubated for 10 min at room temperature and centrifuged at 14,000 X g for 15 min at 4°C. Following centrifugation, the mixture separates into a lower red phenol-chloroform phase, interphase and the colourless upper aqueous phase. RNA remains exclusively in the aqueous phase whereas DNA and proteins are in the interphase and organic phase. Aqueous phase was transferred in to a fresh tube; 0.8 mL of isopropanol was added and mixed with pipette, kept for 15 min at -20 °C and centrifuged at 20,000 g for 20 min at 4 °C. The supernatant was removed and the RNA pellet was washed with 500 µL cold 75% ethanol and centrifuged at 20,000 g for 10 min at 4 °C. Ethanol was aspirated out and the pellet air-dried by inverting the tube. Finally the RNA pellet was dissolved in 15 µL of DEPC treated water, the ODs measured at 260 and 280 nm and the concentrations and purities of RNAs were calculated. Ratios of absorbances at 260 and 280 nm provided the purities of the mRNAs (ratios of absorbances at 260 nm and 280 nm, Abs₂₆₀/Abs₂₈₀, for pure RNA in the range 1.8-2.1) and [A₂₆₀ X Dilution factor X 40] provided the RNA concentrations in µg/mL. 8 µg RNA was used for first-strand cDNA synthesis using the first-strand cDNA synthesis kit (Super script III, Invitrogen) with random hexamers provided in the kit in a final volume of 20 µL. Briefly, in a PCR tube 8 µL (1µg/µL) of isolated RNA, 1µL of primers and 1µL of dNTP mix were mixed and heated to 65 °C for 5 min and cooled to 4°C on PCR machine. In a separate PCR tube 2 µL of 10x RT buffer, 4 µL of 25 mM MgCl₂, 2 µL of 0.1 M DTT, 1µL of RNAse out (40 U/µL) and 1µL of superscript III are mixed. Solution of both the PCR tubes were mixed and reverse transcription reaction were performed using 10 min at 25 °C, 50 min at 50 °C and 5 min at 85 °C and 30 min at 4 °C. Aliquots (2.5 µL) of the cDNA mixture was used to amplify cDNA for Bcl-2, Bcl-XL, Bax, Pl3k, Caspase-9 and 18S RNA (as a loading control) by PCR using the Platinum® PCR SuperMix (Eppendroff). Amplifying solution contained 2.5 µL of cDNA + 2 µL of mixture of Primers (Forward and Reverse, 50 ng/ µL) + 25.5 µL of PCR superMix. Amplification was stopped at 35 cycles. The temperatures cycles used to amplify above genes were as follows: Initial denaturation step 94 °C (2min), Denaturation step 94 °C (30 sec), Primer annealing step 53 °C (30 sec), Extending step 72 °C (30 sec), Final Extetion temperature 72 °C (5 min). The amplified sequences were resolved on a 2% agarose gel electrophoresis and visualized using 0.1% ethidium bromide under UV light.
Primer Sequences used:
Bcl2-For 5'-TGCCAGGACGTCTCCTCTCAG-3'
Bcl2-Rev 5'-AGGTATGCACCCAGAGTGATGCAG-3'
Bax Forward 5'-GTT TCA TCC AGG ATC GAG CAG-3'
Bax Reverse 5'-CAT CTT CTT CCA GAT GGT GA-3
Bcl-xL- sense 5'-TTTG*AATCC*GCCACCATGTCTCAGAGCAACCGGGAGCTG-3'
Bcl-xL-antisense 5'-TTTC*TCGAG*CTTTCCGACTGAAGAGTGAGCCCA-3'
Caspase 3-Forward: CAGTGGAGGCCGACTTCTTG
Caspase 3-Reverse: TGGCACAAAGCGACTGGAT
Caspase 9 - sense 5'-CGGAAGCGGACTGAGGCGGC-3'
Caspase 9 - antisense 5'-CCAATGTCCACTGGTCTGG-3'
18S- Forward: GCAATTATTCCCCATGAACG
18S- Reverse: GGCCTCACTAAACCATCCAA

Results: The significantly decreased mRNA levels of Bcl-2 & Bcl-xL were observed when the cells were treated with liposomes of HD-**16** & HE-**16**. However the other two lipids HD-**18** & HE-**18** did not show significant down regulation of Bcl-2 & Bcl-xL genes. Interestingly, lipids HD-**16**, HD-**18**, HE-**16** & HE-**18** showed up regulation in the expression of pro-apoptotic gene BAX as well as up regulation of Caspase 3 & Caspase 9. 18S m-RNA was used as loading control for all these lanes (Figure 11). Taken together, the findings summarized in Figure 11 demonstrate that several apoptotic signaling pathways get activated upon incubating cancer cells with the liposomal formulations of the presently described histidinylated cationic amphiphiles.

### EXAMPLE 9

### Tumour growth inhibition assay

6-8 weeks old female C57BL/6 mice (each weighing 20-22 g) were purchased from from NIN, Hyderabad, India and kept in our institutional animal house maintaining institutional animal guide line. 1 x 10⁵ B16F1 cells in 100 µL Hank's buffer salt solution were injected subcutaneously with a syringe attached with 30 gauge neddle in the right flank of mice on day 0. On day 14, when the tumors were visible, mice were randomly sorted into six groups and each group (n=5) was administered 150 µM/mouse of liposomal formulations of histidinylated lipids and dexamethasone containing DOPC and a helper lipid Q16TG (with 1:1:1 mole ratio of histidinylated lipid:DOPC:Q16TG) or only DOPC and helper lipid Q16TG (containing 1:1 mole ratio, as a control) at tumour site on day 14, 16, 18, 21 and 24. Tumor volumes (V = 1/2.ab² where, a = maximum length of the tumour and b = minimum length of the tumour measured perpendicular to each other) were measured with a slide calipers for up to 27 days. Results represent the means +/- SD (*P < 0.01 vs HD16 and **P <0.005 vs control).

Results: As depicted in Figure 12 (Parts **A** & **B**), significant tumor growth inhibitions were observed with liposomal formulations of the histidinylated cationic amphiphiles and dexamethasone compared to tumor growth inhibition in control untreated mice. However HD-**16** & HE-**16** showed the highest tumor growth inhibition characteristics; liposomal formuations of HD-**18**, HE-**18** & Dexamethasone showed moderate tumour growth inhibition (Figure 12). Toward confirming that the presently disclosed histidinylated lipids, and not the other liposomal ingredients, play crucial role in mediating observed tumour growth inhibition property, liposomes of only DOPC and helper lipid Q16TG (containing 1:1 mole ratio) combination was used as control. Importantly, such control liposomal formulation containing only Q16TG & DOPC didn't show any tumor growth inhibition effect (Figure 12).

### EXAMPLE 10

### TUNEL assays

6-8 weeks old female C57BL/6 mice (each weighing 20-22 g) were purchased from from NIN, Hyderabad, India and kept in our institutional animal house maintaining institutional animal guide line. 1 x 10⁵ B16F1 cells in 100 µL Hank's buffer salt solution were injected subcutaneously with a syringe attached with 30 gauge neddle in the right flank of mice on day 0. When the mean tumour volume reached - 2000 mm³, At this point, liposomal formulations of the histidinylated cationic amphiphiles and only liposomes of only DOPC and helper lipid Q16TG as a control were intratumourally injected into mice containing aggressive B16 tumours for three consecutive days. Mice were sacrificed 24 h post third day's injection and the tumor were excised, cryosectioned in 5 µm thickness, fixed in 4% formaldehyde and the fixed frozen sections were stained with Promega TUNEL Assay kit according to manufacturer's protocol for marking apoptotic cells. Subsequently, the same tumor crysections were immunounostained with anti-VE-cadherin monoclonal antibody (endothelial cell marker, Satna Cruz) followed by texas-red attached secondary antibody treatment to identify tumor vasculatures. The anti-VE-cadherin monoclonal antibody (1:100 in PBS, 100 µl/section) was added to the same slide for 2 hours followed by washing with PBS (3 x 10 mL). Anti-secondary antibody attached to texas red (1:200 in PBS, 100 µl/section) was added to the slide and incubated for 1 hour. Finally the slides were washed with PBS (3 x10 mL) and air dried. In a vertical fluorescence microscope (100 magnification), the slides were placed and the DNA fragmentations were visualized in green field and the endothelial marker were visualized in red field.

Results: Significant amount of both green (TUNEL positive) and red (endothelial cell positive) fluorescence were visualized under a fluorescence microscope (Figure 13, Parts **A** & **B** respectively). Interestingly the merged panel C (mixing of Part A & B) in figure 13 shows presence of yellow, green and red colours. Presence of yellow colour (due to mixing of green and red colors) confirmed apoptosis in tumour endothelial cells. Abundance of some free green fluorescently labeled cells confirmed simultaneous apoptosis of tumour cells. These findings are consistence with the notion that the liposomes of the presently disclosed histidinylated cationic amphiphiles are capable of inducing apoptosis in both tumor cells and tumor endothelial cells.

Following patents are examples on the development of anti-cancer compounds reported in the past:
1. shaw, et al. cis-platin and folic acid administrated to treat breast cancer. U.S. Pat no. 6,297,245 October 2, 2001
2. Backel, et al. methods for inhibiting angiogenesis proliferation of endothelial or tumor growth. U.S. Pat. No. 5,843,925 December 1, 1999.
3. Brooks, et al. methods and composition useful for inhibition of angiogenesis. U.S. Pat. No.5, 753,230. May 19, 1998.
4. Danter, et al. inhibitor compounds and cancer treatment methods. U.S. Pat. No. 8,138,191. March 20, 2012.

### ADVANTAGES OF THE INVENTION

(1) The histidinylated lipids described in the present invention show better cytotoxicity than those of similar liposomal formulations of the commercially available hydrophobic anti-cancer drug, e.g. dexamethasone in cancerous cells.
(2) The liposomes of the presently disclosed histidinylated cationic amphiphiles are capable of inducing apoptosis in both tumor cells and tumor endothelial cells.
(3) The presently disclosed compounds are useful in treating various types of cancers including lung, melanoma, breast, colon, ovarian cancers, etc.
(4) The compounds can be used in combination therapy with various other anti-cancer drugs or genes.
(5) Significant tumor growth inhibitions are achievable with liposomal formulations of the presently described histidinylated cationic amphiphiles.

## Claims

1. A histidinylated cationic amphiphile compound of formula I 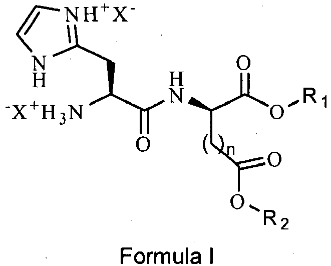 wherein
R1 and R2 are each independently hydrogen or a lipophilic moiety provided both R1 and R2 are not hydrogen at the same time;
X is chlorine or bromine;
n is selected from 1-2 ;
Wherein said lipophilic moiety is selected from the group consisting of C₈₋₂₄ alkyl, monounsaturated, diunsaturated and triunsaturated alkenyl.

2. A compound as claimed in claim 1, wherein the compound is selected from group consisting of compound of formula A and B 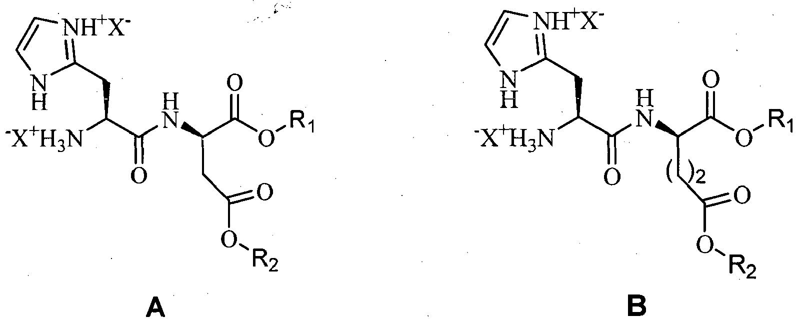 wherein,
R1 and R2 are each independently hydrogen or a lipophilic moiety provided both R1 and R2 are not hydrogen at the same time;
X is chlorine or bromine;
Wherein said lipophilic moiety is selected from the group consisting of C₈₋₂₄ alkyl, monounsaturated, diunsaturated and triunsaturated alkenyl.

3. A compound as claimed in claim 1, wherein the compound is selected from the group consisting of:
(i):2-((S)-2-ammonio-3-((R)-1,4-bis(hexadecyloxy)-1,4-dioxobutan-2-ylamino)-3-oxopropyl)-1H-imidazol-3-iumchloride (HD16),
(ii):2-((S)-2-ammonio-3-((R)-1,4-bis(octadecyloxy)-1,4-dioxobutan-2-ylamino)-3-oxopropyl)-1H-imidazol-3-iumchloride (HD18),
(iii):2-((S)-2-ammonio-3-((R)-1,5-bis(hexadecyloxy)-1,5-dioxopentan-2-ylamino)-3-oxopropyl)-1 H-imidazol-3-iumchloride (HE16), and
(iv):2-((S)-2-ammonio-3-((R)-1,5-bis(octaadecyloxy)-1,5-dioxopentan-2-ylamino)-3-oxopropyl)-1 H-imidazol-3-iumchloride (HE18).

4. The compound as claimed in claim 1, wherein the compound induces apoptosis and inhibits angiogenesis in both tumor endothelial cell and tumor cells.

5. The compound as claimed in claim 1, wherein the compound produces cytotoxic effect in cancer cells at concentration ranging between 10 to 17 micromolar.

6. The compound as claimed in claim 1, wherein the compound inhibits bCL-2 and NF-kB based cell survival pathways.

7. The compound as claimed in claim 1, wherein the compound inhibits 70-90% tumour growth at a dose ranging between 3.0 to 3.5 mg/kg.

8. A process for the preparation of compound of formula I, the process comprising of following steps;
(a) coupling of aliphatic alcohol with N Boc protected derivatives in the presence of a coupling agent and racemization suppressing agent in a polar aprotic solvent, followed by purification to obtain compound of formula II; 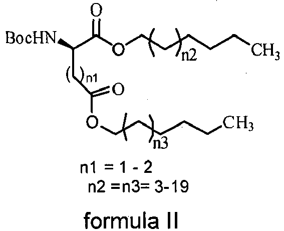
(b) deprotecting the compound of formula II obtained from step (a) using TFA (trifluoro acetic
acid) as deprotecting agent and filtration to obtain a compound of formula III; 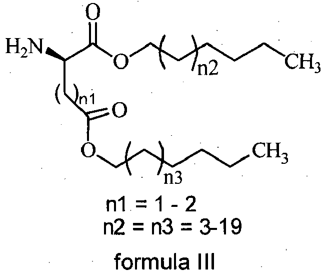
(c) adding N Boc protected histidine to a compound of formula III obtained from step (b) in presence of a racemization suppressing agent and coupling agent in a polar aprotic solvent,
followed by purification to obtain a compound of formula IV ; and 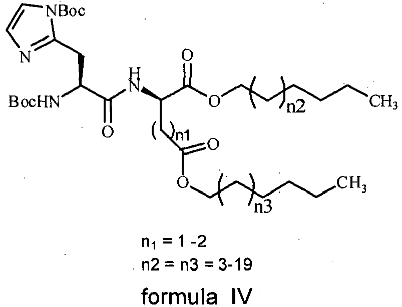
(d) reacting the compound of formula IV obtained from step (c) in presence of a polar aprotic solvent and TFA as deprotecting agent followed by ion exchange chromatography to obtain the compound of formula I.

9. The process as claimed in claim 8, wherein the aliphatic alcohol is selected from the group consisting of saturated or unsaturated alcohol having 8-24 carbon atoms.

10. The process as claimed in claim 8, wherein the polar aprotic solvent is selected from the group consisting of dichloromethane, dimethyl formamide, dimethylsulphoxide, pyridine and triethyl amine.

11. The process as claimed in claim 8 wherein the coupling agent is selected from the group consisting of EDCI (1-ethyl-3-(3-dimethylaminopropyl) carbodiimide) and DCC (1,3-Dicyclohexyl carbodiimide).

12. The process as claimed in claim 8, wherein the racemization suppressing agent is selected from the group consisting of HOBt and HOAt.

13. The process as claimed in claim 8, wherein the N Boc protected derivative is selected from the group consisting of aspartic acid and glutamic acid.

14. A liposomal formulation comprising the histidinylated cationic amphiphile of formula I, a co-lipid and a polyanionic compound optionally along with physiological acceptable additive.

15. The formulation as claimed in claim 14, wherein the cationic amphiphile is either alone or in combination with helper lipids.

16. The formulation as claimed in claim 14, wherein the co-lipid is selected from the group consisting of phosphatidylethanolamine, phosphatidylphosphocholine, phosphatidylglycerol, cholesterol. 1,2-syn-dioleyolglycerophosphoethanolamine (DOPE) and 1,2-syndioleyolglycerophosphocholine (DOPC).

17. The formulation as claimed in claim 15, wherein the helper lipid is *N*,*N*-Di-*n*-hexadecyl-*N-*[2-guanidinyl]ethyl-*N*-methylammonium Chloride (Q16TG).

18. The formulation as claimed in clam 15, wherein the molar ratio of cationic amphiphiles:co-lipid:helper lipid ranges from 1:1:1 to 3:2:1.

19. The formulation as claimed in claim 14, wherein the polyanionic compound is an anionic synthetic phospholipid.

20. The formulation as claimed in claim 14, wherein the physiological acceptable additive is selected from the group comprising of saline and 5 % glucose solution.

21. The formulation as claimed in claim 14, wherein the formulation exhibits cellular cytotoxicity when the amount of cationic amphiphile used is in the range of 2 to 30 micro molar concentration.

22. A method for preparation of liposomal formulation, the method comprising of following steps;
a. dissolving a histidinylated cationic amphiphile compound of formula I, a co-lipid, and a helper lipid in the mole ratio ranging from 1:1:1 to 3:2:1 in a mixture of methanol and chloroform followed by evaporation of the solvent in presence of a thin flow of moisture free nitrogen gas to obtain lipid film;
b. drying the lipid film obtained from step (a) under vacuum;
c. hydrating the dried lipid film in sterile deionized water;and
d. vortexing the resulting mixture obtained from previous step to remove any adhering lipid film, sonicating the vortexed mixture in a bath sonicator at room temperature to produce multilamellar vesicles (MLVs) and sonicating the resulting MLVs with Ti-probe sonicator to produce clear translucent small unilamellar vesicles (SUVs).

23. The method as claimed in claim 22, wherein the co lipid is selected from the group comprising phosphatidylethanolamine, phosphatidylphosphocholine, phosphatidylglycerol, cholesterol. 1,2-syn-dioleyolglycerophosphoethanolamine (DOPE) and 1,2-syndioleyolglycerophosphocholine (DOPC).

24. The method as claimed in claim 22, wherein the helper lipid is *N*,*N*-Di-*n*-hexadecyl-*N*-[2-guanidinyljethyl-*N*-methylammonium Chloride (Q16TG).

25. A method as claimed in claim 22 wherein the ratio of chloroform and methanol used in step (a) ranges from 1:1 to 4:1.

## Patentansprüche

1. Histidinylierte kationische amphiphile Verbindung der Formel I 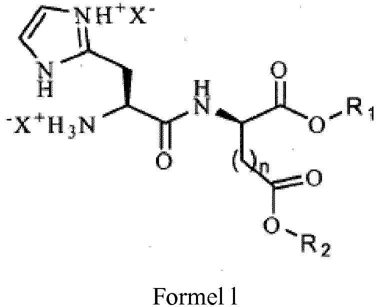 wobei
R1 und R2 unabhängig voneinander Wasserstoff oder eine lipophile Einheit sind, vorausgesetzt, dass R1 und R2 nicht gleichzeitig Wasserstoff sind;
X für Chlor oder Brom steht;
N aus 1 bis 2 ausgewählt ist;
wobei die lipophile Einheit aus der Gruppe ausgewählt wird, die aus Folgendem besteht: C₈₋₂₄-Alkyl, einfach ungesättigtem, zweifach ungesättigtem und dreifach ungesättigtem Alkenyl.

2. Verbindung nach Anspruch 1, wobei die Verbindung aus der Gruppe ausgewählt wird, die aus einer Verbindung der Formel A und B besteht 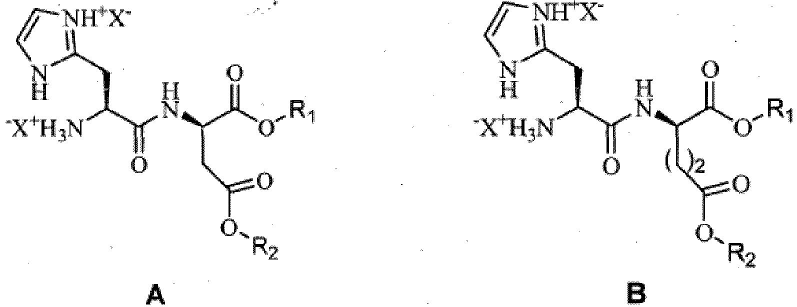 wobei
R1 und R2 unabhängig voneinander Wasserstoff oder eine lipophile Einheit sind, vorausgesetzt, dass R1 und R2 nicht gleichzeitig Wasserstoff sind;
X für Chlor oder Brom steht;
wobei die lipophile Einheit aus der Gruppe ausgewählt wird, die aus Folgendem besteht: C₈₋₂₄-Alkyl, einfach ungesättigtem, zweifach ungesättigtem und dreifach ungesättigtem Alkenyl.

3. Verbindung nach Anspruch 1, wobei die Verbindung aus der Gruppe ausgewählt wird, die aus Folgendem besteht:
(i): 2-((S)-2-Ammonium-3-((R)-1,4-bis(hexadecyloxy)-1,4-dioxobutan-2-ylamino)-3-oxopropyl)-1H-imidazol-3-iumchlorid (HD16),
(ii): 2-((S)-2-Ammonium-3-((R)-1,4-bis(octadecyloxy)-1,4-dioxobutan-2-ylamino)-3-oxopropyl)-1H-imidazol-3-iumchlorid (HD18),
(iii): 2-((S)-2-Ammonium-3-((R)-1,5-bis(hexadecyloxy)-1,5-dioxopentan-2-ylamino)-3-oxopropyl)-1H-imidazol-3-iumchlorid (HE16) und
(iv): 2-((S)-2-Ammonium-3-((R)-1,5-bis(octadecyloxy)-1,5-dioxopentan-2-ylamino)-3-oxopropyl)-1H-imidazol-3-iumchlorid (HE18).

4. Verbindung nach Anspruch 1, wobei die Verbindung Apoptose induziert und die Angiogenese sowohl in der Tumorendothelzelle als auch in den Tumorzellen hemmt.

5. Verbindung nach Anspruch 1, wobei die Verbindung eine zytotoxische Wirkung in Krebszellen bei einer Konzentration im Bereich von 10 bis 17 Mikromol erzeugt.

6. Verbindung nach Anspruch 1, wobei die Verbindung die bCL-2- und NF-kBbasierten Zellüberlebenswege hemmt.

7. Verbindung nach Anspruch 1, wobei die Verbindung 70 bis 90 % des Tumorwachstums bei einer Dosis zwischen 3,0 und 3,5 mg/kg hemmt.

8. Verfahren zur Herstellung der Verbindung der Formel I, wobei das Verfahren folgende Schritte umfasst:
(a) Koppeln von aliphatischem Alkohol mit N-Boc-geschützten Derivaten in Gegenwart eines Kopplungsmittels und eines Racemisierungsunterdrückungsmittels in einem polaren aprotischen Lösungsmittel, gefolgt von einer Reinigung, um eine Verbindung der Formel II zu erhalten; 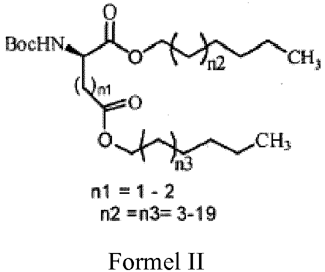
(b) Entschützen der Verbindung der Formel II, die aus Schritt (a) erhalten wurde, unter Verwendung von TFA (Trifluoressigsäure) als Entschützungsmittel und Filtration, um eine Verbindung der Formel III zu erhalten; 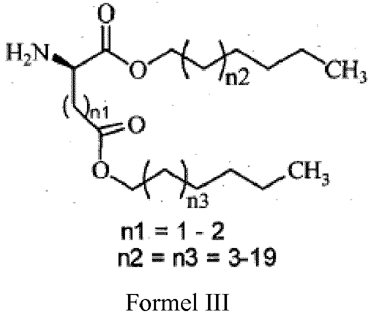
(c) Zugabe von N-Boc-geschütztem Histidin zu einer Verbindung der Formel III, die aus Schritt (b) erhalten wurde, in Gegenwart eines Racemisierungsunterdrückungsmittels und eines Kopplungsmittels in einem polaren aprotischen Lösungsmittel, gefolgt von einer Reinigung, um eine Verbindung der Formel IV zu erhalten; und 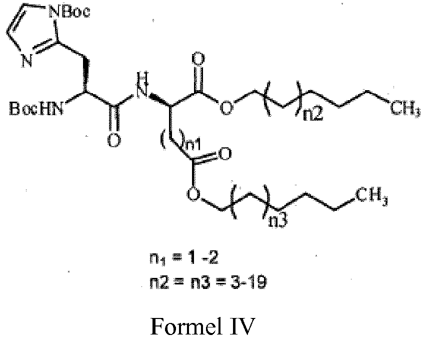
(d) Reagieren der Verbindung der Formel IV, die aus Schritt (c) erhalten wurde, in Gegenwart eines polaren aprotischen Lösungsmittels und TFA als Entschützungsmittel gefolgt von einer Ionenaustauschchromatographie, um die Verbindung der Formel I zu erhalten.

9. Verfahren nach Anspruch 8, wobei der aliphatische Alkohol aus der Gruppe ausgewählt wird, die aus gesättigtem oder ungesättigtem Alkohol mit 8 bis 24 Kohlenstoffatomen besteht.

10. Verfahren nach Anspruch 8, wobei das polare aprotische Lösungsmittel aus der Gruppe ausgewählt wird, die aus Folgendem besteht: Dichlormethan, Dimethylformamid, Dimethylsulfoxid, Pyridin und Triethylamin.

11. Verfahren nach Anspruch 8, wobei das Kopplungsmittel aus der Gruppe ausgewählt wird, die aus EDCI (1-Ethyl-3-(3-dimethylaminopropyl) carbodiimid) und DCC (1,3-Dicyclohexylcarbodiimid) besteht.

12. Verfahren nach Anspruch 8, wobei das Racemisierungsunterdrückungsmittel aus der Gruppe ausgewählt wird, die aus HOBt und HOAt besteht.

13. Verfahren nach Anspruch 8, wobei das N-Boc-geschützte Derivat aus der Gruppe ausgewählt wird, die aus Asparaginsäure und Glutaminsäure besteht.

14. Liposomale Formulierung, die Folgendes umfasst: das histidinylierte kationische Amphiphil der Formel I, ein Co-Lipid und eine polyanionische Verbindung, optional zusammen mit einem physiologisch akzeptablen Additiv.

15. Formulierung nach Anspruch 14, wobei das kationische Amphiphil entweder allein oder in Kombination mit Helferlipiden vorliegt.

16. Formulierung nach Anspruch 14, wobei das Co-Lipid aus der Gruppe ausgewählt wird, die aus Folgendem besteht: Phosphatidylethanolamin, Phosphatidylphosphocholin, Phosphatidylglycerol, Cholesterin, 1,2-Syn-Dioleyolglycerophosphoethanolamin (DOPE) und 1,2-Syn-Dioleyolglycerophosphocholin (DOPC).

17. Formulierung nach Anspruch 15,
wobei das Helferlipid *N*,*N*-Di-*n*-hexadecyl-*N*-[2-guanidinyl]ethyl-*N-*methylammoniumchlorid (Q16TG) ist.

18. Formulierung nach Anspruch 15,
wobei das Molverhältnis von kationischen Amphiphilen zu Co-Lipid zu Helferlipid im Bereich von 1:1:1 bis 3:2:1 liegt.

19. Formulierung nach Anspruch 14, wobei die polyanionische Verbindung ein anionisches synthetisches Phospholipid ist.

20. Formulierung nach Anspruch 14, wobei das physiologisch akzeptable Additiv aus der Gruppe ausgewählt wird, die Kochsalzlösung und 5 % Glucoselösung umfasst.

21. Formulierung nach Anspruch 14, wobei die Formulierung zelluläre Zytotoxizität aufweist, wenn die eingesetzte Menge an kationischem Amphiphil im Bereich einer Konzentration von 2 bis 30 Mikromol liegt.

22. Verfahren zur Herstellung einer liposomalen Formulierung, wobei das Verfahren folgende Schritte umfasst:
a. Auflösen einer histidinylierten kationischen amphiphilen Verbindung der Formel I, eines Co-Lipids und eines Helferlipids im Molverhältnis im Bereich von 1:1:1 bis 3:2:1 in einem Gemisch aus Methanol und Chloroform gefolgt von Verdampfen des Lösungsmittels in Gegenwart eines dünnen Stroms von feuchtigkeitsfreiem Stickstoffgas, um Lipidfolie zu erhalten;
b. Trocknen der aus Schritt (a) erhaltenen Lipidfolie unter Vakuum;
c. Hydratisieren der getrockneten Lipidfolie in sterilem deionisiertem Wasser und
d. Verwirbeln der resultierenden Mischung, die aus dem vorherigen Schritt erhalten wurde, um anhaftende Lipidfolie zu entfernen, Beschallen der verwirbelten Mischung in einem Badbeschallungsgerät bei Raumtemperatur, um multilamellare Vesikel (MLVs) zu erzeugen, und Beschallen der resultierenden MLVs mit einem Ti-Sondensonicator, um klare durchscheinende kleine unilamellare Vesikel (SUVs) zu erzeugen.

23. Verfahren nach Anspruch 22, wobei das Co-Lipid aus der Gruppe ausgewählt wird, die aus Folgendem besteht: Phosphatidylethanolamin, Phosphatidylphosphocholin, Phosphatidylglycerol, Cholesterin, 1,2-Syn-Dioleyolglycerophosphoethanolamin (DOPE) und 1,2-Syn-Dioleyolglycerophosphocholin (DOPC).

24. Verfahren nach Anspruch 22, wobei das Helferlipid *N*,*N*-Di-*n*-hexadecyl-*N*-[2-guanidinyl]ethyl-*N*-methylammoniumchlorid (Q16TG) ist.

25. Verfahren nach Anspruch 22, wobei das in Schritt (a) verwendete Verhältnis von Chloroform und Methanol im Bereich von 1:1 bis 4:1 liegt.

## Revendications

1. Composé amphiphile cationique histidinylé de la formule I: 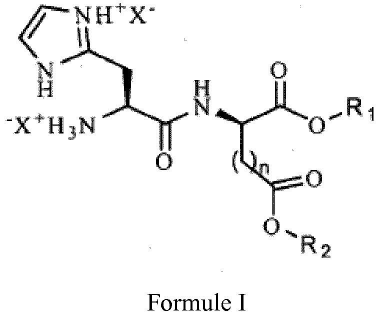 dans lequel:
R1 et R2 sont chacun indépendamment un hydrogène ou une fraction lipophile à condition que les deux R1 et R2 ne soient pas un hydrogène en même temps;
X est un chlore ou un brome;
n est choisi parmi 1-2;
dans lequel ladite fraction lipophile est choisie dans le groupe constitué d'un C₈₋₂₄ alkyle, alcényle mono-insaturé, di-insaturé et tri-insaturé.

2. Composé selon la revendication 1, où le composé est choisi dans le groupe constitué d'un composé des formules A et B: 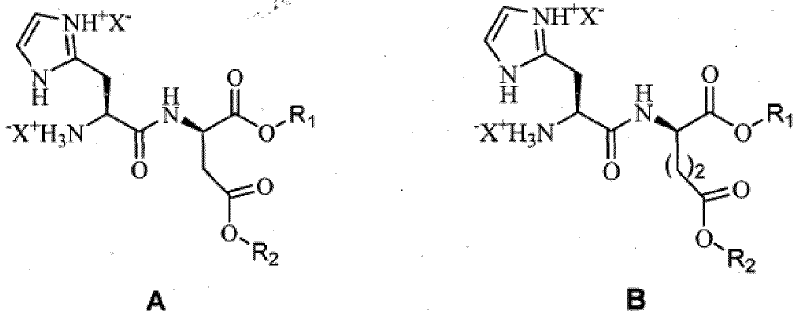 dans lequel:
R1 et R2 sont chacun indépendamment un hydrogène ou une fraction lipophile à condition que les deux R1 et R2 ne soient pas un hydrogène en même temps;
X est un chlore ou un brome;
dans lequel ladite fraction lipophile est choisie dans le groupe constitué d'un C₈₋₂₄ alkyle, alcényle mono-insaturé, di-insaturé et tri-insaturé.

3. Composé selon la revendication 1, où le composé est choisi dans le groupe constitué de:
(i): chlorure de 2-((S)-2-ammonio-3-((R)-1,4-bis(hexadécyloxy)-1,4-dioxobutan-2-ylamino)-3-oxopropyl)-1H-imidazol-3-ium (HD16),
(ii): chlorure de 2-((S)-2-ammonio-3-((R)-1,4-bis(octadécyloxy)-1,4-dioxobutan-2-ylamino)-3-oxopropyl)-1H-imidazol-3-ium (HD18),
(iii): chlorure de 2-((S)-2-ammonio-3-((R)-1,5-bis(hexadécyloxy)-1,5-dioxopentan-2-ylamino)-3-oxopropyl)-1H-imidazol-3-ium (HE16), et
(iv): chlorure de 2-((S)-2-ammonio-3-((R)-1,5-bis(octadécyloxy)-1,5-dioxopentan-2-ylamino)-3-oxopropyl)-1H-imidazol-3-ium (HE18).

4. Composé selon la revendication 1, où le composé induit une apoptose et inhibe une angiogenèse dans à la fois une cellule endothéliale tumorale et des cellules tumorales.

5. Composé selon la revendication 1, où le composé produit un effet cytotoxique dans des cellules cancéreuses à une concentration variant entre 10 et 17 micromolaire.

6. Composé selon la revendication 1, où le composé inhibe des voies de survie de cellules à base de bCL-2 et Nf-kB.

7. Composé selon la revendication 1, où le composé inhibe 70-90% de croissance tumorale à une dose variant entre 3,0 et 3,5 mg/kg.

8. Procédé pour la préparation d'un composé de la formule I, le procédé comprenant les étapes suivantes:
(a) couplage d'un alcool aliphatique avec des dérivés protégés par N Boc en présence d'un agent de couplage et d'un agent de suppression de racémisation dans un solvant aprotique polaire, suivi par une purification pour obtenir un composé de la formule II: 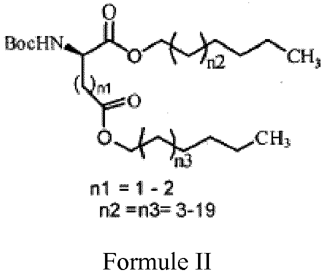
(b) déprotection du composé de la formule II obtenu à partir de l'étape (a) en utilisant TFA (acide trifluoroacétique) en tant qu'agent de déprotection et filtration pour obtenir un composé de la formule III: 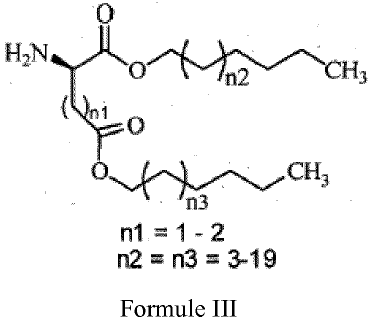
(c) ajout d'histidine protégée par N Boc au composé de la formule III obtenu à partir de l'étape (b) en présence d'un agent de suppression de racémisation et d'un agent de couplage dans un solvant aprotique polaire, suivi par une purification pour obtenir un composé de la formule IV; et 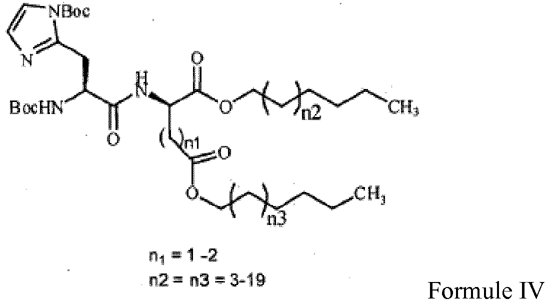
(d) réaction du composé de la formule IV obtenu à partir de l'étape (c) en présence d'un solvant aprotique polaire et de TFA en tant qu'agent de déprotection suivi par une chromatographie d'échange d'ions pour obtenir le composé de la formule I.

9. Procédé selon la revendication 8, dans lequel l'alcool aliphatique est choisi dans le groupe constitué d'un alcool saturé ou insaturé possédant 8-24 atomes de carbone.

10. Procédé selon la revendication 8, dans lequel le solvant aprotique polaire est choisi dans le groupe constitué de dichlorométhane, diméthylformamide, diméthylsulfoxyde, pyridine et triéthylamine.

11. Procédé selon la revendication 8, dans lequel l'agent de couplage est choisi dans le groupe constitué de EDCI (1-éthyl-3-(3-diméthylaminopropyl)carbodiimide) et DCC (1,3-dicyclohexylcarbodiimide).

12. Procédé selon la revendication 8, dans lequel l'agent de suppression de racémisation est choisi dans le groupe constitué de HOBt et HOAt.

13. Procédé selon la revendication 8, dans lequel le dérivé protégé par N Boc est choisi dans le groupe constitué d'acide aspartique et d'acide glutamique.

14. Formulation de liposomes comprenant l'amphiphile cationique histidinylé de la formule I, un co-lipide et un composé polyanionique optionnellement accompagné d'un additif physiologique acceptable.

15. Formulation selon la revendication 14, dans laquelle l'amphiphile cationique est soit seul, soit en combinaison avec des lipides auxiliaires.

16. Formulation selon la revendication 14, dans laquelle le co-lipide est choisi dans le groupe constitué de phosphatidyléthanolamine, phosphatidylphosphocholine, phosphatidylglycérol, cholestérol, 1,2-syn-dioléyolglycérophosphoéthanolamine (DOPE) et 1,2-syn-dioléyolglycérophosphocholine (DOPC).

17. Formulation selon la revendication 15, dans laquelle le lipide auxiliaire est le chlorure de *N*,*N*-di-*n*-hexadécyl-*N*-[2-guanidinyl]éthyl-*N*-méthylammonium (Q16TG).

18. Formulation selon la revendication 15, dans laquelle le rapport molaire d'amphiphiles cationiques:co-lipide:lipide auxiliaire varie de 1:1:1 à 3:2:1.

19. Formulation selon la revendication 14, dans laquelle le composé polyanionique est un phospholipide synthétique anionique.

20. Formulation selon la revendication 14, dans laquelle l'additif physiologique acceptable est choisi dans le groupe comprenant une solution saline et une solution de glucose à 5%.

21. Formulation selon la revendication 14, où la formulation affiche une cytotoxicité cellulaire lorsque la quantité d'amphiphile anionique utilisée est dans l'intervalle de concentration de 2 à 30 micromolaire.

22. Méthode pour la préparation d'une formulation de liposomes, la méthode comprenant les étapes suivantes:
a. dissolution d'un composé amphiphile cationique histidinylé de la formule I, d'un co-lipide et d'un lipide auxiliaire dans le rapport molaire variant de 1:1:1 à 3:2:1 dans un mélange de méthanol et de chloroforme suivi par l'évaporation du solvant en présence d'un écoulement mince d'azote gazeux sans humidité pour obtenir un film lipidique;
b. séchage du film lipidique obtenu à partir de l'étape (a) sous vide;
c. hydratation du film lipidique séché dans de l'eau déminéralisée stérile; et
d. agiter au vortex le mélange résultant obtenu à partir de l'étape précédente pour retirer tout film lipidique adhérant, passage aux ultrasons du mélange agité au vortex dans un bac à ultrasons à la température ambiante pour produire des vésicules multi-lamellaires (MLVs) et passage aux ultrasons des MLVs résultantes avec un générateur d'ultrasons à sonde Ti pour produire des petites vésicules uni-lamellaires translucides claires (SUVs).

23. Méthode selon la revendication 22, dans laquelle le co-lipide est choisi dans le groupe comprenant la phosphatidyléthanolamine, la phosphatidylphosphocholine, le phosphatidylglycérol, le cholestérol, la 1,2-syn-dioléyolglycérophosphoéthanolamine (DOPE) et la 1,2-syn-dioléyolglycérophosphocholine (DOPC).

24. Méthode selon la revendication 22, dans laquelle le lipide auxiliaire est le chlorure de *N*,*N*-di-*n*-hexadécyl-*N*-[2-guanidinyl]éthyl-*N*-méthylammonium (Q16TG).

25. Méthode selon la revendication 22, dans laquelle le rapport du chloroforme et du méthanol utilisé dans l'étape (a) varie de 1:1 à 4:1.
